(19) Europäisches Patentamt
European Patent Office
Office européen des brevets

(11) EP 3 622 954 A1

(12) **EUROPEAN PATENT APPLICATION**
published in accordance with Art. 153(4) EPC

(43) Date of publication:
**18.03.2020 Bulletin 2020/12**

(21) Application number: **18798031.3**

(22) Date of filing: **07.05.2018**

(51) Int Cl.:
***A61K 31/444** (2006.01)* ***A61P 25/14** (2006.01)*
***A61P 43/00** (2006.01)* ***C07D 491/147** (2006.01)*

(86) International application number:
**PCT/JP2018/017619**

(87) International publication number:
**WO 2018/207729 (15.11.2018 Gazette 2018/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.05.2017 JP 2017093173**
**09.05.2017 JP 2017093174**
**09.05.2017 JP 2017093175**

(71) Applicant: **Eisai R&D Management Co., Ltd.**
**Tokyo 112-8088 (JP)**

(72) Inventors:
- **IDO, Katsutoshi**
**Tsukuba-shi**
**Ibaraki 300-2635 (JP)**
- **WAKITA, Hisashi**
**Tsukuba-shi**
**Ibaraki 300-2635 (JP)**
- **HATANAKA, Ken**
**Tsukuba-shi**
**Ibaraki 300-2635 (JP)**

(74) Representative: **Hoffmann Eitle**
**Patent- und Rechtsanwälte PartmbB**
**Arabellastraße 30**
**81925 München (DE)**

(54) **THERAPEUTIC AGENT CONTAINING PYRANODIPYRIDINE COMPOUND**

(57) A compound represented by formula (I) or a pharmaceutically acceptable salt thereof has potential use as an agent for treating essential tremor, small fiber neuropathy, dystonia, epilepsy associated with Alzheimer's disease, epilepsy associated with brain tumor, or brain tumor.

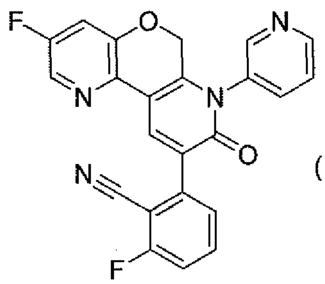

(I)

EP 3 622 954 A1

## Description

### Technical Field

The present invention relates to agents for treating essential tremor, small fiber neuropathy, dystonia, epilepsy associated with Alzheimer's disease, epilepsy associated with brain tumor, or brain tumor, comprising a pyranodipyridine compound having $\alpha$-amino-3-hydroxy-5-methyl-4-isoxazolepropionic acid (AMPA) receptor inhibitory action or a pharmaceutically acceptable salt thereof as an active ingredient.

### Background Art

Essential tremor is a pathological condition which presents only tremor primarily in posturing and acting, without causal factors including pre-existing diseases, organic abnormality, traumas, and medication history. Essential tremor develops in many cases at around 40 years old, and is exacerbated by aging and persists for the lifetime. The frequency of the onset increases with age. With respect to the onset of essential tremor, essential tremor found with a familial trend is often transmitted in autosomal dominant inheritance and called familial tremor; and essential tremor which has developed in an elderly person is called senile tremor.

While drug therapy is primarily performed for essential tremor, the only drug within insurance coverage for essential tremor with evidence of efficacy is propranolol, which is recommended as a first-choice drug by a therapeutic guideline (see Non Patent Literature 1). Patients with complications of, for example, asthma, bradycardia, diabetes mellitus, cardiac failure, or pheochromocytoma have, however, contraindications for propranolol. Although the guideline also recommends the anti-epileptic drug primidone as a first-choice drug, primidone causes side effects including drowsiness and sedation. Because of problems with respect to the effect or side effects, 56.3% of patients discontinue taking such a first-choice drug, and use an anti-epileptic drug or the like such as clonazepam, topiramate, gabapentin, and pregabalin as an adjuvant (see Non Patent Literature 2). These drugs are, however, not completely proved to have efficacy and may require use of multiple drugs or drug switching, and in addition suffer from a problem of development of side effects including bradycardia, hypotension, dizziness, sedation, weight gain, fatigue, drowsiness, ataxia, and nausea, and thus there is currently no sufficiently satisfactory treatment agent (see Non Patent Literature 3). Alcohol is known to mitigate symptoms of essential tremor, and long-term use thereof causes a high risk of alcoholism (see Non Patent Literature 4).

In spite of the situation that there are many existing drugs as described above, patients having difficulty in daily life and significant reduction in QOL are known (see Non Patent Literature 5), and development of a drug effective for essential tremor has been demanded.

While the pathological mechanism of essential tremor is still unclear, essential tremor is believed to be one of central tremors. Animals to which harmaline has been administered are used as an animal model of essential tremor. Tremor of 4 to 12 Hz in acting is induced by harmaline, and the tremor is attenuated with a drug for treatment of essential tremor. Harmaline has action of inducing coincident firing of neuronal activities of inferior olivary nuclei, and this is expected to be the cause of generation of tremor (see Non Patent Literature 6).

Although a previous report says that 2,3-dioxo-6-nitro-1,2,3,4-tetrahydrobenzo[f]quinoxaline-7-sulfonamide (NBQX), an antagonist for an AMPA receptor and a kainate receptor, suppresses tremor in a harmaline animal model (see Non Patent Literature 7), no pyranodipyridine compound having inhibitory action on tremor is known.

Small fiber neuropathy (SFN) is a new disease concept referring to a group with impairment primarily of small fibers among peripheral neuropathies.

Peripheral nerves include somatic nerves consisting of motor nerves and sensory nerves, and an automatic nervous system to involuntarily control functions of organs in the body, and a condition with these nerves impaired is called peripheral neuropathy. It has been reported that 2.4% of the general population and 8% of persons 55 years or older are affected, and thus there exist an extremely large number of patients (Non Patent Literatures 8, 9). Sensory nerves and automatic nerves are roughly classified by the presence or absence of a myelin sheath, the thickness of a fiber, and difference in conduction velocity into A$\beta$, A$\delta$, and C fibers (Non Patent Literature 10). A$\beta$ fibers are each a fiber having a myelin sheath and a large diameter, and involved in rapid nerve conduction and primarily transmit tactile sensation and pressure sensation. A$\delta$ fibers each have a myelin sheath and a diameter smaller than those of A$\beta$ fibers, and transmit relatively rapid thermal sensation and pain sensation. C fibers are each a fiber lacking a myelin sheath and having a small diameter, and transmit sustained, slow visceral pain and temperature in sensory nerves and control automatic nerves in postganglionic autonomic fibers.

Conduction velocity is mainly measured in tests on peripheral neuropathy, but this approach can primarily check only the function of large A$\beta$ fibers. Through recent progress of diagnostic techniques, however, methods for detecting impairment of small fibers (A$\delta$ and C fibers) with skin biopsies have been becoming common. In association with this, SFN is proposed as a disease concept indicating that primarily small fibers are impaired among peripheral neuropathies,

and has been becoming common.

SFN is caused by various underlying diseases such as metabolic diseases including diabetes mellitus, infection by HIV and so on, amyloidosis, drug- or poison-induced diseases, and idiopathic diseases (Non Patent Literature 11). Abnormality of the sensory system such as pain, burning sensation, tingling sensation, and anaesthesia is found for SFN patients (Non Patent Literature 11). When small fibers in automatic nerves are impaired, dry eye, thirst, orthostatic dizziness, sudomotor dysfunction, and so on are found (Non Patent Literature 12). The QOL of such a patient is largely lowered in mental and physical senses by those symptoms, and in particular pain strongly affects QOL (Non Patent Literature 13), and hence removal of pain is an important goal of treatment.

There are very few clinical studies conducted for SFN, and neither a drug with strong evidence of analgesic effect nor an analgesic within insurance coverage for SFN is present. In treatment of SFN, pain is controlled by using tricyclic antidepressants, serotonin-norepinephrine uptake inhibitors or anticonvulsants, opioid analgesics, and so on, which are not within insurance coverage but used for treatment of neuralgia. It has been reported, however, that analgesics generally provide only 20 to 30% pain reduction and only 20 to 35% of patients undergo 50% pain relief, and effect of analgesics for SFN is strongly desired (Non Patent Literature 14).

A streptozotocin (STZ)-induced model is known as an SFN animal model (Non Patent Literature 15). It has been previously reported that fever and hyperalgesia to mechanical stimulation are suppressed through direct administration of the AMPA/kainate receptor inhibitor 6-cyano-7-nitroquinoxaline-2,3-dione (CNQX) to the anterior cingulate cortex in the forebrain in STZ models (Non Patent Literature 16); however, no pyranodipyridine compound having analgesic action is known.

Dystonia is defined as "a pathological state in which a specific posture or movement is disturbed by repeated, twisting, persistent muscle contractions with a constant pattern".

Dystonia is roughly classified by pathological condition into two types (Non Patent Literature 17). Primary dystonia refers to cases that dystonia is the only or essential cardinal symptom and any of the known independent disease units cannot be identified as the causal pathological condition. Secondary dystonia refers to dystonia caused as a symptom of a known independent disease or caused by an external factor. Examples of the external factor include drugs, poisons, and traumas. Examples of drug-induced dystonia include acute dystonia and delayed dystonia which appear as a side effect of a drug. There is secondary dystonia caused by neurological diseases such as Parkinsonian syndrome and Wilson disease.

Dystonia is classified by the site into local dystonia (for example, blepharospasm dystonia, cervical dystonia, writer's cramp dystonia, pharyngeal dystonia), multifocal dystonia (dystonia presenting as dystonia at multiple sites not adjacent to each other), systemic dystonia (coincidence of segmental dystonia in a leg and dystonia at another site), and segmental dystonia (cases of dystonia at adjacent multiple sites which does not meet the definition of systemic dystonia).

Since dystonia is fatal if being systemic, and involves lowering of QOL including interference with movements in daily life such as postures and frequently-used movements, influence on his or her occupation, and reduction in frequency of going-out due to worrying about his or her deteriorated posture, various treatments are performed for dystonia (Non Patent Literature 18).

Botulinum treatment is used as a first choice for treatment of local dystonia (Non Patent Literature 19). Botulinum treatment is selected even for systemic dystonia if muscle relaxation at a specific site is determined to lead to improvement of QOL or determined to be effective for preventing progression of complications. In the case of systemic dystonia, deep brain stimulation (DBS) is performed, for example, for the internal globus pallidus (Non Patent Literature 20). Even during botulinum therapy or DBS, an oral medicine of an anticholinergic, clonazepam, diazepam, baclofen, or the like is used as adjuvant therapy (Non Patent Literature 20).

While various treatment methods have been tested and symptoms have been controlled as described above, there are still many cases with insufficient effect. Botulinum treatment requires injection in hospitals and thus is inconvenient, and in addition what is acceptable is only use of specified doses for specified muscles within insurance coverage (Non Patent Literature 20). There exist dystonia for which DBS is recommended, such as primary systemic dystonia, but DBS requires surgery to place stimulating electrodes in the brain, and hence there is strong need for a simple alternative therapy.

While the pathological mechanism of dystonia is unclear in many points, abnormal activity of the basal ganglia, which is involved in motor function control, is inferred to be the cause. As a dystonia model, the hamster $dt^{sz}$, which undergoes spontaneous onset of dystonia-like symptoms, is known (Non Patent Literature 21). It has been previously reported that the AMPA receptor inhibitor 2,3-dioxo-6-nitro-1,2,3,4-tetrahydrobenzo[f]quinoxalme-7-sulfonamide (NBQX) has anti-dystonia action in the $dt^{sz}$ model (Non Patent Literature 21); however, no pyranodipyridine compound having anti-dystonia action is known.

The epilepsy incidence rate in Alzheimer's disease (AD) has been reported to be about seven times higher than that in non-dementia cases (Non Patent Literature 22). In addition, abnormal electroencephalograms without the occurrence of convulsions, called subclinical epileptiform activity, have been found for 42% of AD cases in earlier stages

of the disease (Non Patent Literature 23). Further, a high epilepsy incidence rate of 48% is found on average for early-onset familial AD cases, and about 81% for cases with a specific genotype (APP duplication) (Non Patent Literature 24). These results suggest that AD is a risk factor of epilepsy.

On the other hand, there are reports suggesting that epilepsy is a risk factor of AD. The following two reports are the representative examples: (1) lowering of cognitive functions is more rapid for AD patients having subclinical epileptiform activity than for AD patients having no subclinical epileptiform activity (Non Patent Literature 23); and (2) adults who underwent the onset of epilepsy in childhood have increased amyloid accumulation in the brain (Non Patent Literature 25). Thus, AD and epilepsy are related to each other.

In the human AD brain, expression of a subunit constituting calcium-permeable AMPA receptors such as GLUR1 and GLUR2 (Q) is facilitated (Non Patent Literatures 26, 27). Amyloid-beta (Aβ), which is believed to be deeply involved in progression of pathological condition of AD, enhances expression of GluR1 on cell surfaces in the hippocampus of rats, and increases the excitatory postsynaptic current mediated by an AMPA receptor (Non Patent Literature 28). If an AMPA receptor inhibitor is applied in advance, on the other hand, the increase of the excitatory postsynaptic current by Aβ does not occur (Non Patent Literature 28). These results suggest that expression and hyperactivity of an AMPA receptor is a cause of the onset of epilepsy in AD.

As an AD-related epilepsy model, AD model mice including a Tg2576 mouse and a 5XFAD mouse are known, and each of them has been reported to spontaneously exhibit a an electroencephalogram with abnormal excitation (Non Patent Literatures 29, 30). Currently, there is no report showing that an AMPA receptor inhibitor exhibits anticonvulsant action in those models.

Brain tumor is roughly classified into two types, namely, primary brain tumor, which is generated from the brain, and metastatic brain tumor, which is generated through metastasis of cancer generated in tissue or an organ other than the brain. In contrast to other cancers, neither TMN classification nor staging is used for brain tumors, and classification is made on the basis of 1 to 4 levels of malignancy (grades). Most benign brain tumors correspond to Grade 1, and representative examples thereof include intracranial meningioma, pituitary adenoma, and neurilemmoma. Representative examples of malignant brain tumors include glioma.

Neurons are supported by neuloglial cells. The collective name for tumors generated from neuloglial cells is glioma, which accounts for about 25% of tumors generated from the brain. Glioma is further classified into multiple types. Representative examples thereof are: astrocytoma and oligodendroglioma (all Grade 2); anaplastic astrocytoma, anaplastic oligodendroglioma, and anaplastic mixed oligodendroglioma-astrocytoma (all Grade 3); and glioblastoma (Grade 4).

In standard treatment of glioma in an incipient stage, tumor is resected as much as possible, and radiotherapy and chemotherapy with temozolomide are simultaneously performed for the residual tumor. Because glioma invades the brain tissue to grow, however, it is difficult to completely resect glioma by surgery, and glioma often recurs. The five-year survival rate is 50 to 70% for Grade 2, 20 to 33% for Grade 3, and 8% or lower for Grade 4, and thus glioma is a disease with poor prognosis. In addition, epilepsy develops with a high probability, resulting in lowered QOL. In particular, epilepsy develops in about 70% of glioma cases of low grade, namely, Grades 1 and 2 (Non Patent Literature 31). Regarding the type of seizure, serious seizures including tonic-clonic seizure, complex partial seizure, and secondary generalized seizure are mainly found, convulsions do not disappear in 66% of glioma cases even when three or more anti-epileptic drugs are used in combination, and thus glioma is a disease with high unmet need (Non Patent Literature 32).

As a glioma-related epilepsy model, a model is known which is obtained by intracranially transplanting a patient-derived glioma cell or a human glioma cell line to a nude mouse (Non Patent Literature 33). Currently, no AMPA receptor inhibitor which exhibits anticonvulsant action in this model is known. It has been reported that the AMPA receptor inhibitor YM872 has antitumor effect in a model obtained by subcutaneously transplanting a human glioma cell line to a nude mouse (Non Patent Literature 34); however, no pyranodipyridine compound which exhibits antitumor effect in this model is known.

## Citation List

### Non Patent Literature

[Non Patent Literature 1] Neurology, 64, p2008-2020 (2005)
[Non Patent Literature 2] Parkinsonism and Related Disorders, 16, p604-607(2010)
[Non Patent Literature 3] Journal of Central Nervous System Disease, 5, p43-55 (2013)
[Non Patent Literature 4] The American Journal of Medicine, 115, p134-142 (2003)
[Non Patent Literature 5] Journal of Neurological Sciences, 349, p168-173 (2015)
[Non Patent Literature 6] Journal of Physiology, 376, p163-182 (1986)

[Non Patent Literature 7] European Journal of Pharmacology, 616, p73-80 (2009)
[Non Patent Literature 8] Martyn CN et al, (1997) Neurol Neurosurg Psychiatry Epidemiology of peripheral neuropathy
[Non Patent Literature 9] Hughes RC et al (2002)Br Med J 324: 466-469 Peripheral Neuropathy
[Non Patent Literature 10] Basbaum AI et al. Cellular and molecular mechanisms of pain. Cell. (2009) 139(2): 267-84
[Non Patent Literature 11] Chan AC and Wilder-Smith EP.Small fiber neuropathy: Getting bigger! Muscle Nerve (2016) 53(5): 671-82.
[Non Patent Literature 12] Tavee J and Zhou L. (2009) Cleve Clin J Med. 76(5): 297-305.
[Non Patent Literature 13] Bakkers M et al. Small fibers, large impact: quality of life in small-fiber neuropathy. Muscle Nerve. (2014) 49(3): 329-36
[Non Patent Literature 14] Nanna Brix Finnerup et al. Chapter 17-Management of painful neuropathies. Handbook of Clinical Neurology (2013) 115,279-290
[Non Patent Literature 15] Viktor R Drel et al. Evaluation of the peroxynitrite decomposition catalyst Fe(III) tetramesitylporphyrin octasulfonate on peripheral neuropathy in a mouse model of type 1 diabetes. (2007) Int J Mol Med. 20(6): 783-792
[Non Patent Literature 16] Tomiyama M et al. Upregulation of mRNAs coding for AMPA and NMDA receptor subunits and metabotropic glutamate receptors in the dorsal horn of the spinal cord in a rat model of diabetes mellitus. Brain Res Mol Brain Res (2005) 20; 136(1-2): 275-281
[Non Patent Literature 17] Bressman SB: Dystonia genotypes, phenotypes, and classification. Adv Neurol 2004 (94) 101-107
[Non Patent Literature 18] Page D et al: Quality of life in focal, segmental, and generalized dystonia, Mov Disord 2007, 22(3), 341-347
[Non Patent Literature 19] E. Zoons et al: Botulinum toxin as treatment for focal dystonia: a systematic review of the pharmaco-therapeutic and pharmaco-economic value. Journal of Neurology 2012, 259(12), 2519-2526
[Non Patent Literature 20] Termsarasab P et al: Medical treatment of dystonia, J Clin Move Disorder 2016,3(19)
[Non Patent Literature 21] RichterA et al: The AMPA receptor antagonist NBQX exerts antidystonic effects in an animal model of idiopathic dystonia 1993, 231, 287-291
[Non Patent Literature 22] Horvath A et al: Epileptic seizures in Alzheimer disease: A review. Alzheimer Dis. Assoc. Disord. 2016, 30(2), 186-192
[Non Patent Literature 23] Vossel KA et al: Incidence and impact of subclinical epileptiform activity in Alzheimer's disease. Ann. Neurol. 2016, 80(6), 858-870
[Non Patent Literature 24] Zarea A et al: Seizures in dominantly inherited Alzheimer disease. Neurology, 2016, 87(9), 912-919
[Non Patent Literature 25] Joutsa J et al: Association between childhood-onset epilepsy and amyloid burden 5 decades later. JAMANeurol. 2017, 74(5), 583-590
[Non Patent Literature 26] Marcello E et al: SAP97-mediated local trafficking is altered in Alzheimer disease patients' hippocampus. Neurobiol. Aging, 2012, 33(2), 422.e1-10
[Non Patent Literature 27] Gaisler-Salomon I et al: Hippocampus-specific deficiency in RNAediting of GluA2 in Alzheimer's disease. Neurobiol. Aging, 2014,35(8), 1785-1791
[Non Patent Literature 28] Whitcomb DJ et al: Intracellular oligomeric amyloid-beta rapidly regulates GluA1 subunit ofAMPA receptor in the hippocampus. Sci. Rep. 2015, 5,10934
[Non Patent Literature 29] Bezzina C et al: Early onset of hypersynchronous network activity and expression of a marker of chronic seizures in the Tg2576 mouse model of Alzheimer's disease. PLoS One, 2015, 10(3), e0119910
[Non Patent Literature 30] Siwek ME et al: Altered theta oscillations and aberrant cortical excitatory activity in the 5XFAD model of Alzheimer's disease. Neural Plast. 2015, 2015, 781731
[Non Patent Literature 31] Li Y et al: IDH1 mutation is associated with a higher preoperative seizure incidence in low-grade glioma: A systematic review and meta-analysis. Seizure 2018, 55, 76-82
[Non Patent Literature 32] Neal A et al: Postoperative seizure control in patients with tumor-associated epilepsy. Epilepsia 2016, 57(11), 1779-1788
[Non Patent Literature 33] Buckingham SC et al: Glutamate release by primary brain tumors induces epileptic activity. Nat Med. 2011, 17(10), 1269-1274
[Non Patent Literature 34] Ishiuchi S et al: Blockage of Ca2+-permeable AMPA receptors suppresses migration and induces apoptosis in human glioblastoma cells. Nat. Med 2002, 8(9), 971-978

## Summary of Invention

### Technical Problem

An object of the present invention is to provide a compound or pharmaceutically acceptable salt thereof which

exhibits antitremor action in animal models and has potential use as an agent for treating essential tremor, exhibits anti-SFN action in animal models and has potential use as an agent for treating SFN, exhibits anti-dystonia action in animal models and has potential use as an agent for treating dystonia, exhibits anticonvulsant action against epilepsy associated with Alzheimer's disease in animal models and has potential use as an agent for treating epilepsy associated with Alzheimer's disease, exhibits anticonvulsant action against epilepsy associated with brain tumor in animal models and has potential use as an agent for treating epilepsy associated with brain tumor, or exhibits antitumor action against brain tumor in animal models and has potential use as an agent for treating brain tumor.

**Solution to Problem**

The present inventors have continued vigorous research to achieve the above-described object, and consequently have found that pyranodipyridine compounds or pharmaceutically acceptable salts thereof have effects of suppressing tremor induced by harmaline in a rat harmaline model and have effects of suppressing pain induced by STZ in a rat STZ model, their effects of suppressing dystonia can be confirmed in a $dt^{sz}$ hamster model, their effects of suppressing convulsions can be confirmed in an AD model mouse such as a Tg2576 mouse and a 5XFAD mouse, their effects of suppressing convulsions can be confirmed in a mouse model with intracranially transplanted human glioma cells, and their antitumor effects can be confirmed in a mouse model with subcutaneously transplanted human glioma cells, thereby completing the present invention.

In summary, the present invention relates to <1> to <53> set forth below.

<1> An agent for treating essential tremor, small fiber neuropathy, dystonia, epilepsy associated with Alzheimer's disease, epilepsy associated with brain tumor, or brain tumor, comprising a compound selected from the group consisting of:

2-auoro-6-(3-nuoro-8-oxo-7-(pyndin-3-yl)-7,8-dihydro-6H-pyrano[3,2-b:5,4-b']dipyridin-9-yl)benzonitrile,

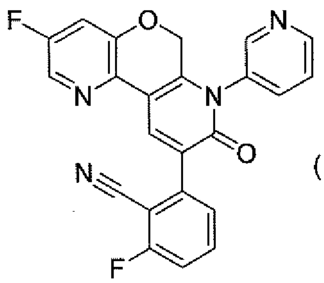

(I)

2-fluoro-6-(8-oxo-7-(pyrimidin-5-yl)-7,8-dihydro-6H-pyrano[3,2-b:5,4-b']dipyridin-9-yl)benzonitrile,

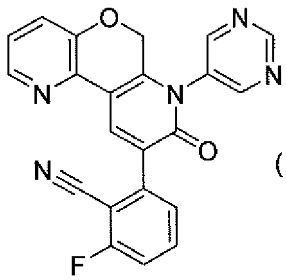

(II)

and

2-fluoro-6-(7-(2-methylpyrimidin-5-yl)-8-oxo-7,8-dihydro-6H-pyrano[3,2-b:5,4-b']dipyridin-9-yl)benzonitrile

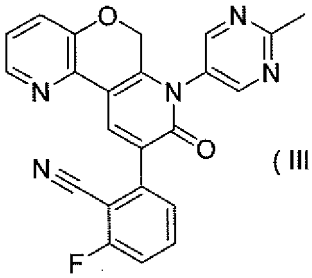

( III )

or a pharmaceutically acceptable salt thereof.

<2> An agent for treating essential tremor, small fiber neuropathy, dystonia, epilepsy associated with Alzheimer's disease, or epilepsy associated with brain tumor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

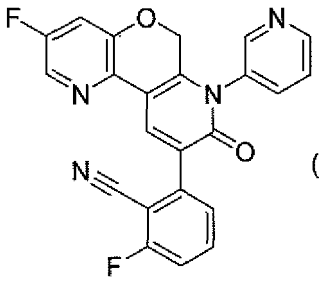

( I )

or a pharmaceutically acceptable salt thereof.
<3> An agent for treating essential tremor or small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzomtrile represented by formula (I):

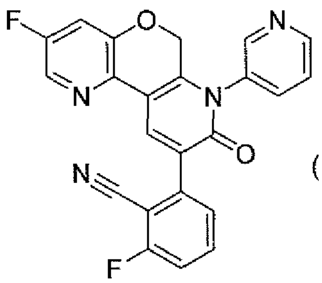

( I )

or a pharmaceutically acceptable salt thereof.
<4> A method for treating essential tremor or small fiber neuropathy, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

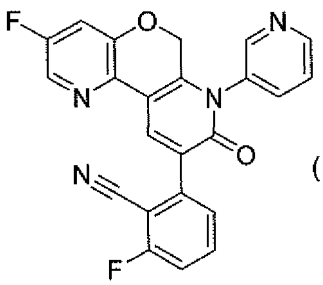

( I )

or a pharmaceutically acceptable salt thereof.
<5> 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

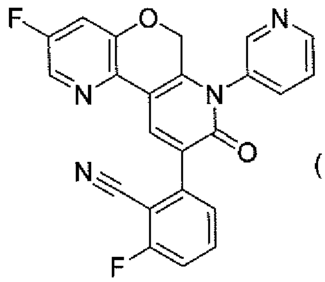

( I )

or a pharmaceutically acceptable salt thereof for use in treating essential tremor or small fiber neuropathy.

<6> Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

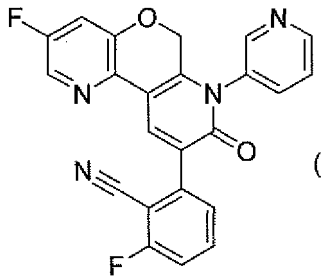

( I )

or a pharmaceutically acceptable salt thereof in the manufacture of an agent for treating essential tremor or small fiber neuropathy.

<7> A composition for essential tremor or small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

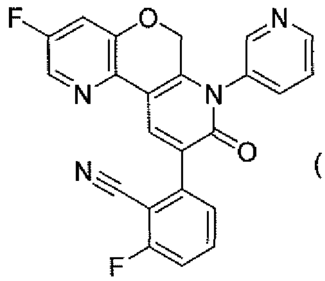

( I )

or a pharmaceutically acceptable salt thereof.

<8> A pharmaceutical composition for treating essential tremor or small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

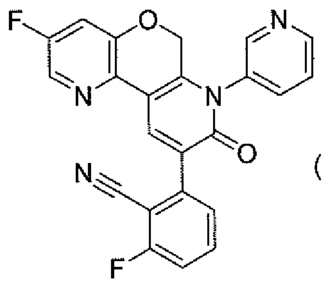

( I )

or a pharmaceutically acceptable salt thereof.

<9> The pharmaceutical composition according to <8>, further comprising a pharmaceutically acceptable excipient.

<10> An agent for treating essential tremor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

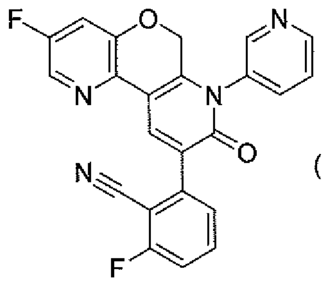

(I)

or a pharmaceutically acceptable salt thereof.

<11> A method for treating essential tremor, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

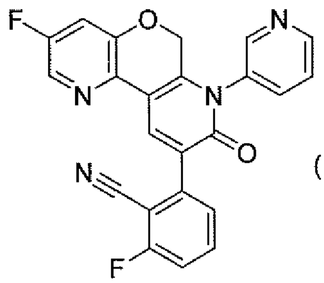

(I)

or a pharmaceutically acceptable salt thereof.

<12> 2-Fluoro-6-(3-fluoro-8-oxo-7-(pyridin-3-yl)-7,8-dihydro-6H-pyrano[3,2-b:5,4-b']dipydin-9-yl)benzonitrile represented by formula (I):

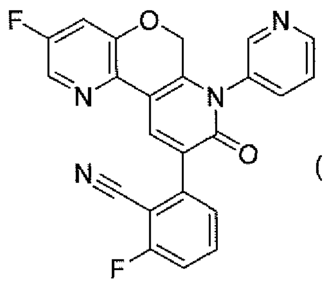

(I)

or a pharmaceutically acceptable salt thereof for use in treating essential tremor.

<13> Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

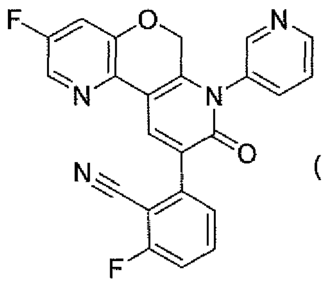

(I)

or a pharmaceutically acceptable salt thereof in the manufacture of an agent for treating essential tremor.

<14> A composition for essential tremor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

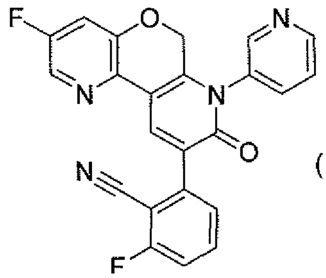

(I)

or a pharmaceutically acceptable salt thereof.

<15> A pharmaceutical composition for treating essential tremor, comprising 2-fluoro-6-(3-fluoro-8-oxo-7-(pyridin-3-yl)-7,8-dihydro-6H-pyrano[3,2-b:5,4-b']dipyndin-9-yl)benzonitrile represented by formula (I):

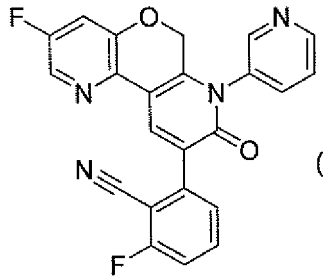

(I)

or a pharmaceutically acceptable salt thereof.

<16> The pharmaceutical composition according to <15>, further comprising a pharmaceutically acceptable excipient.

<17> An agent for treating small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

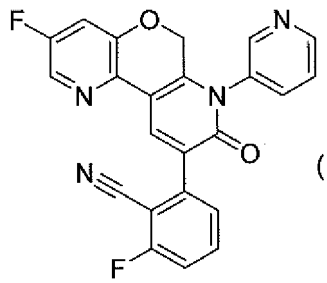

(I)

or a pharmaceutically acceptable salt thereof.

<18> A method for treating small fiber neuropathy, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

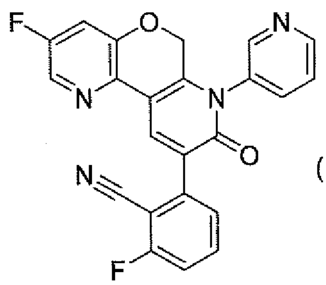

(I)

or a pharmaceutically acceptable salt thereof.

<19> 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

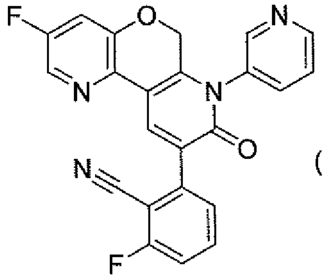

(I)

or a pharmaceutically acceptable salt thereof for use in treating small fiber neuropathy.

<20> Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

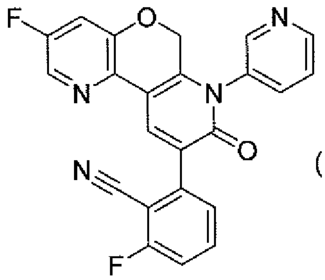

(I)

or a pharmaceutically acceptable salt thereof for the manufacture of an agent for treating small fiber neuropathy.

<21> A composition for small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

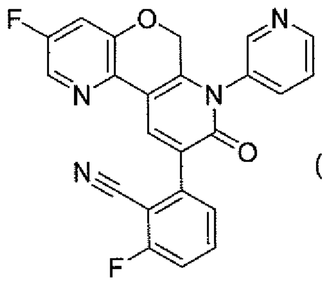

(I)

or a pharmaceutically acceptable salt thereof.

<22> A pharmaceutical composition for treating small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

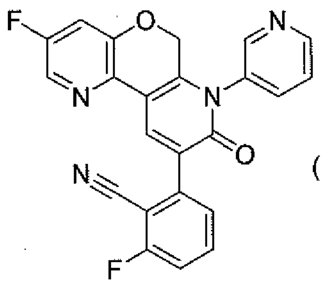

(I)

or a pharmaceutically acceptable salt thereof.

<23> The pharmaceutical composition according to <22>, further comprising a pharmaceutically acceptable excipient.

<24> An agent for treating dystonia, comprising 2-fluoro-6-(3-fluoro-8-oxo-7-(pyridin-S-yl)-7,8-dihydro-6H-pyrano[3,2-b:5,4-b']dipyridin-9-yl)benzonitrile represented by formula (I):

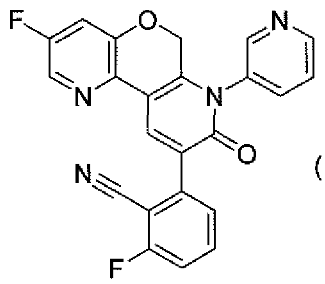

( I )

or a pharmaceutically acceptable salt thereof.

<25> A method for treating dystonia, comprising administering to a patient in need thereof 2-fluoro-6-(3-fluoro-8-oxo-7-(pyridin-3-yl)-7,8-dihydro-6H-pyrano[3,2-b:5,4-b']diridin-9-yl)benzonitrile represented by formula (I):

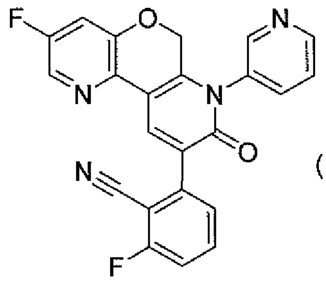

( I )

or a pharmaceutically acceptable salt thereof.

<26> 2-Ffluoro-6-(3-fluoro-8-oxo-7-(pyridin-3-yl)-7,8-dihydro-6H-pyrano[3,2-b:5,4-b']dipyridin-9-yl)benzonitile represented by formula (I):

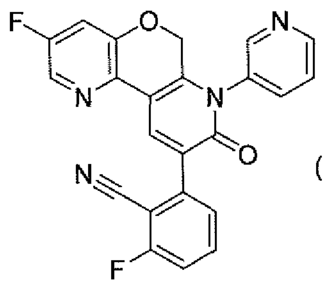

( I )

or a pharmaceutically acceptable salt thereof for use in treating dystonia.

<27> Use of 2-fluoro-6-(3-fluoro-8-oxo-7-(pyridin-3-yl)-7,8-dihydro-6H-pyrano[3,2-b:5,4-b']dipydin-9-yl)benzonitrile represented by formula (I):

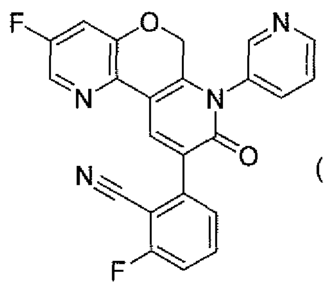

( I )

or a pharmaceutically acceptable salt thereof in the manufacture of an agent for treating dystonia.

<28> A pharmaceutical composition for treating dystonia, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

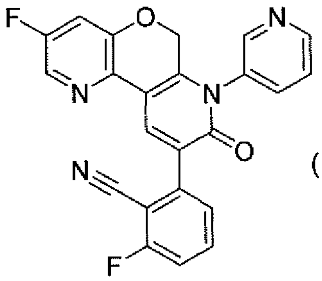

(I)

or a pharmaceutically acceptable salt thereof.

<29> A pharmaceutical composition for treating dystonia, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

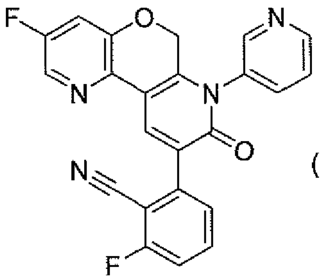

(I)

or a pharmaceutically acceptable salt thereof.

<30> The pharmaceutical composition according to <29>, further comprising a pharmaceutically acceptable excipient.

<31> An agent for treating epilepsy associated with Alzheimer's disease, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

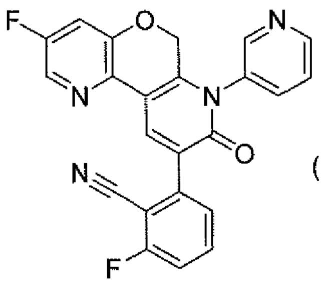

(I)

or a pharmaceutically acceptable salt thereof.

<32> A method for treating epilepsy associated with Alzheimer's disease, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

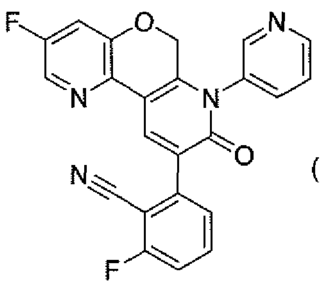

(I)

or a pharmaceutically acceptable salt thereof.

<33> 2-Fluoro-6-(3-auoro-8-oxo-7-(pyndin-3-yl)-7,8-dihydno-6H-pyrano[3,2-b:5,4-b']dipyridin-9-yl)benzonitrile represented by formula (I):

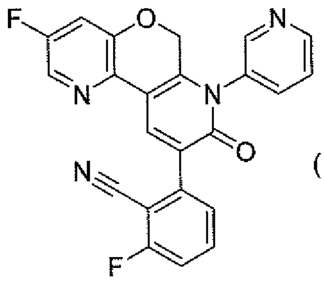

( I )

or a pharmaceutically acceptable salt thereof for use in treating epilepsy associated with Alzheimer's disease.

<34> Use of 2-fluoro-6-(3-fluoro-8-oxo-7-(pyridin-3-yl)-7,8-dihydin-6H-pyrano[3,2-b:5,4-b']dipyridin-9-yl)benzonitrile represented by formula (1):

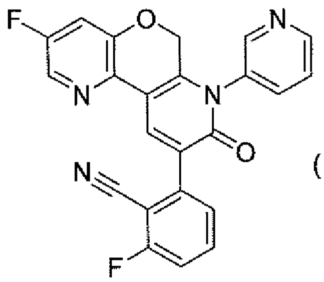

( I )

or a pharmaceutically acceptable salt thereof in the manufacture of an agent for treating epilepsy associated with Alzheimer's disease.

<35> A composition for epilepsy associated with Alzheimer's disease, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

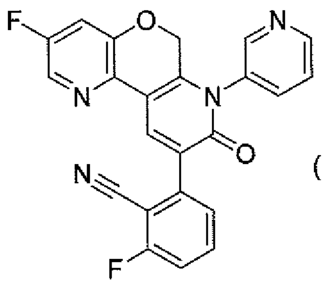

( I )

or a pharmaceutically acceptable salt thereof.

<36> A pharmaceutical composition for treating epilepsy associated with Alzheimer's disease, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

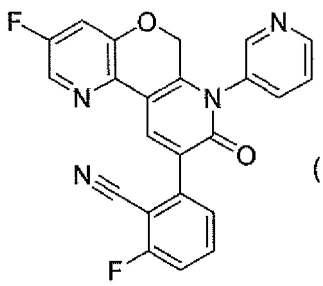

( I )

or a pharmaceutically acceptable salt thereof.

<37> The pharmaceutical composition according to <36>, further comprising a pharmaceutically acceptable excipient.

<38> An agent for treating epilepsy associated with brain tumor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

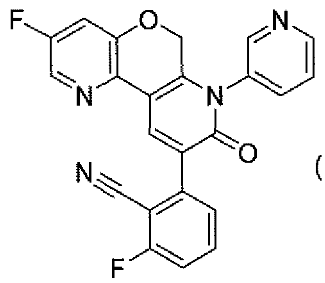

(I)

or a pharmaceutically acceptable salt thereof.

<39> A method for treating epilepsy associated with brain tumor, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

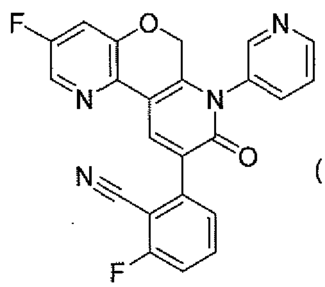

(I)

or a pharmaceutically acceptable salt thereof.

<40> 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

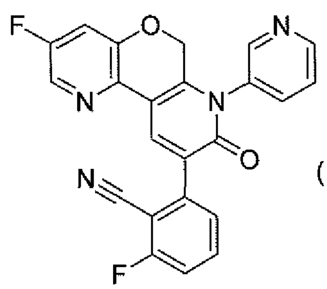

(I)

or a pharmaceutically acceptable salt thereof for use in treating epilepsy associated with brain tumor.

<41> Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

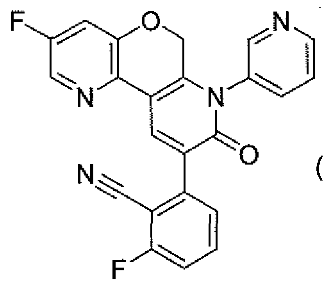

(I)

or a pharmaceutically acceptable salt thereof in the manufacture of an agent for treating epilepsy associated with brain tumor.

<42> A composition for epilepsy associated with brain tumor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

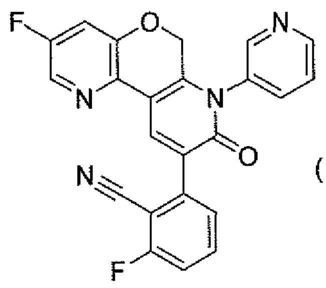

(I)

or a pharmaceutically acceptable salt thereof.

<43> A pharmaceutical composition for treating epilepsy associated with brain tumor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

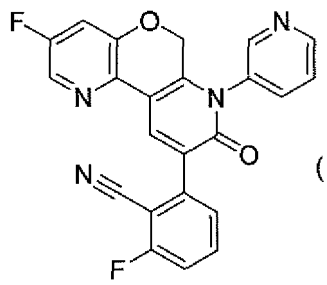

(I)

or a pharmaceutically acceptable salt thereof.

<44> The pharmaceutical composition according to <43>, further comprising a pharmaceutically acceptable excipient.

<45> Any of the treatment agents, treatment methods, compounds, uses, compositions, or pharmaceutical compositions above, wherein the brain tumor is glioma.

<46> An agent for treating brain tumor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

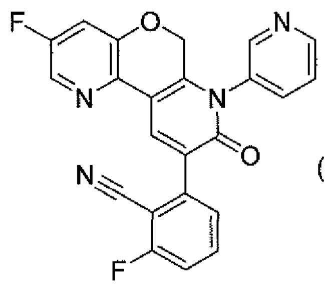

(I)

or a pharmaceutically acceptable salt thereof.

<47> A method for treating brain tumor, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

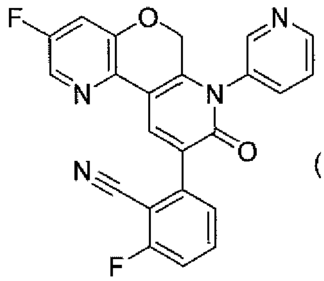

(I)

or a pharmaceutically acceptable salt thereof.

<48> 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

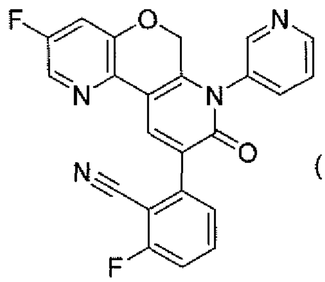

(I)

or a pharmaceutically acceptable salt thereof for use in treating brain tumor.

<49> Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

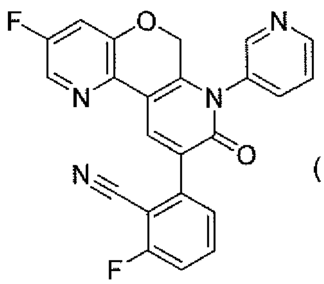

(I)

or a pharmaceutically acceptable salt thereof in the manufacture of an agent for treating brain tumor.

<50> A composition for brain tumor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

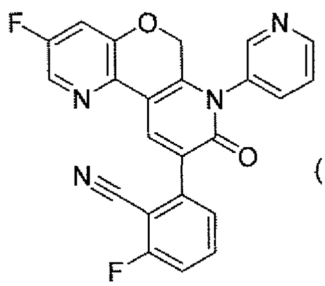

(I)

or a pharmaceutically acceptable salt thereof.

<51> A pharmaceutical composition for treating brain tumor, comprising 2-fluoro-6-(3-fluoro-8-oxo-7-(pyridin-3-yl)-7,8-dihydro-6H-pyrano[3,2-b:5,4-b]dipyridin-9-yl)benzonitrile represented by formula (I):

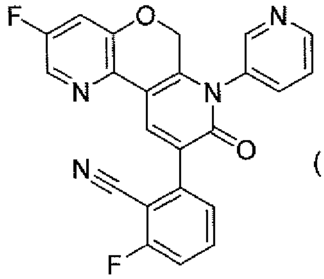

(I)

or a pharmaceutically acceptable salt thereof.

<52> The pharmaceutical composition according to <51>, further comprising a pharmaceutically acceptable excipient.

<53> Any of the treatment agents, treatment methods, compounds, uses, compositions, or pharmaceutical compositions above, wherein the brain tumor is glioma.

**Advantageous Effects of Invention**

The pyranodipyridine compound or a pharmaceutically acceptable salt thereof of the present invention exhibits effect of suppressing tremor induced by harmaline in a rat harmaline model as an animal model of essential tremor, effect of suppressing pain induced by STZ in a rat STZ model as an animal model of small fiber neuropathy, effect of suppressing dystonia in a $dt^{sz}$ hamster model as an animal model of dystonia, effect of suppressing convulsions in a Tg2576 mouse and a 5XFAD mouse as an animal model of Alzheimer's disease, effect of suppressing convulsions in a mouse model with intracranially transplanted human glioma cells as an animal model of brain tumor, or antitumor effect in a mouse model with subcutaneously transplanted human glioma cells as an animal model of brain tumor. Accordingly, the compound or a pharmaceutically acceptable salt thereof according to the present invention has potential use as an agent for treating essential tremor, small fiber neuropathy, dystonia, epilepsy associated with Alzheimer's disease, epilepsy associated with brain tumor, or brain tumor.

**Description of Embodiments**

The present invention will be hereinafter described in detail.

In the compounds of the present specification, the structural formula may represent a certain isomer for convenience sake; however, the compound is not limited to the formula shown for convenience sake, and includes all the structurally possible isomers and isomeric mixtures of the compound, such as geometrical isomers, optical isomers, rotamers, stereoisomers and tautomers, and may be either one of the isomers, or a mixture containing each of the isomers at a given ratio. Thus, optical isomers and a racemate, for example, may be present for the compound in the present specification; however, it is not limited to any of them in the present specification, and the compound in the present specification may be a racemate, any of the optically active substances, or a mixture containing each of the optically active substances at a given ratio.

Further, crystalline polymorphs may also be present, although the present invention is similarly not limited to any of them, and the compound of the present invention may be in a single form of any of the crystal forms, or a mixture thereof, and the present invention also includes amorphous forms. The compound of the present invention also encompasses anhydrate and solvates (in particular, hydrate).

As used herein, the "pharmaceutically acceptable salt" is not particularly limited as long as it is a salt formed with any of the compounds of the present invention, and may specifically be, for example, an acid addition salt such as an inorganic acid salt, an organic acid salt, or an acidic amino acid salt.

With regard to the "pharmaceutically acceptable salt" herein, unless otherwise indicated, the number of molecules of the acid relative to one molecule of the compound in the formed salt is not particularly limited as long as a salt with an appropriate ratio is formed; however, the number of molecules of the acid relative to one molecule of the compound in one mode is about 0.1 to about 5, in another mode about 0.5 to about 2, and in still another mode about 0.5, about 1, or about 2.

Examples of inorganic acid salts in one specific mode include hydrochloride, hydrobromide, sulfate, nitrate, and phosphate, and examples of organic acid salts in one specific mode include acetate, succinate, fumarate, maleate, tartrate, citrate, lactate, stearate, benzoate, methanesulfonate, p-toluenesulfonate, and benzenesulfonate.

Examples of acidic amino acid salts in one specific mode include aspartate and glutamate.

When the pyranodipyridine compounds according to the present invention are obtained in the free form, they can be converted to salts or hydrates thereof that may be formed by the compounds described above, in accordance

with a conventional method.

When the pyranodipyridine compounds according to the present invention are obtained as salts or hydrates of the compounds, they can be converted to the free form of the compounds described above, in accordance with a conventional method.

Moreover, various isomers obtained for the compounds in the present specification (for example, geometrical isomers, optical isomers, rotamers, stereoisomers, and tautomers) can be purified and isolated using common separation means, for example, recrystallization, a diastereomeric salt formation method, an enzymatic resolution method, and various types of chromatography (for example, thin layer chromatography, column chromatography, and gas chromatography).

[Preparation]

A pharmaceutical composition of the invention could be prepared by mixing a pharmaceutically acceptable excipient with a pyranodipyridine compound or a pharmaceutically acceptable salt thereof. A pharmaceutical composition of the invention can be prepared in accordance with the known method such as a method described in the General Rules for Preparations of the Japanese Pharmacopoeia 17th Edition, the United States Pharmacopeia, and the European Pharmacopoeia.

A pharmaceutical composition of the invention could be administered to patients appropriately depending on the dosage form.

The dose of the pyranodipyridine compound or a pharmaceutically acceptable salt thereof according to the present invention will vary depending on the severity of the condition, age, sex, body weight, type of the dosage form or salt, specific type of the disease, and the like; generally, however, for an adult, in the case of oral administration, the daily dose is about 30 μg to 10 g, 100 μg to 5 g in one mode, and 100 μg to 1 g in another mode, and in the case of administration by injection, the daily dose is about 30 μg to 1 g, 100 μg to 500 mg in one mode, and 100 μg to 300 mg in another mode, each administered in single or several divided doses.

[Combination]

The pyranodipyridine compound or a pharmaceutically acceptable salt thereof according to the present invention can be administered as an agent for treating essential tremor to a patient, for example, in combination with primidone, propranolol, alprazolam, gabapentin, sotalol, topiramate, clonazepam, clozapine, or nadolol simultaneously, separately, continuously, or at time intervals. The pyranodipyridine compound or a pharmaceutically acceptable salt thereof according to the present invention can be administered as an agent for treating small fiber neuropathy to a patient, for example, in combination with a tricyclic antidepressant (such as nortriptyline, amitriptyline, imipramine), a Ca channel $\alpha 2\delta$ ligand (such as pregabalin, gabapentin), neurotropin, a serotonin/noradrenaline reuptake inhibitor (such as duloxetine), an antiarrhythmic (such as mexiletine), an aldose reductase inhibitor (such as epalrestat), oxycodone, carbamazepine, lamotrigine, baclofen, tramadol, codeine, fentanyl, morphine, buprenorphine, or pentazocine simultaneously, separately, continuously, or at time intervals. Further, the pyranodipyridine compound or a pharmaceutically acceptable salt thereof according to the present invention can be administered as an agent for treating dystonia to a patient, for example, in combination with botulinum toxin, an anticholinergic (such as trihexyphenidyl), an anti-epileptic drug (such as clonazepam, levetiracetam), an L-dopa preparation, an atypical psychotropic (such as risperidone, clozapine), or a muscle relaxant (such as baclofen) simultaneously, separately, continuously, or at time intervals.

## Examples

The pyranodipyridine compound of the present invention can be produced, for example, using the methods described in the following production examples and referential examples, and the effects of the compounds can be confirmed using the methods described in the following Test Example. It should be noted, however, that these examples are illustrative, and the present invention is in any case not limited to the following specific examples, and modifications may be made thereto without departing from the scope of the present invention.

Compounds for which document names or the like are noted were produced in accordance with the document or the like.

The abbreviations used herein are conventional abbreviations well known to those skilled in the art. The following abbreviations will be used herein:

$(Ataphos)_2PdCl_2$: bis(di-t-butyl(4-dimethylaminophenyl)phosphine)dichloropalladium(II)
DCM: dichloromethane
DMEAD: di-2-methoxyethyl azodicarboxylate

DMF: N,N-dimethylformamide
DMSO: dimethylsulfoxide
EDC: 1-ethyl-3-(3-dimethylaminopropyl)carbodiimide hydrochloride
MTBE: 2-methoxy-2-methylpropane
n-: normal
NBS: N-bromosuccinimide
t-: tertiary
TFA: trifluoroacetic acid
THF: tetrahydrofuran
$^1$H-NMR: proton nuclear magnetic resonance spectrometry
MS: mass spectrometry

In the following production examples and referential examples, the "room temperature" generally refers to about 10°C to about 35°C. "%" refers to percent by weight, unless otherwise specified.

Chemical shifts in proton nuclear magnetic resonance spectra are recorded in δ units (ppm) relative to tetramethylsilane, and coupling constants are recorded in hertz (Hz). Abbreviations for splitting patterns are as follows:
s: singlet, d: doublet, t: triplet, q: quartet, m: multiplet, br.s: broad singlet.

For reactions using a microwave reactor in the production examples and referential examples, initiator™ or initiator+™ from Biotage Corporation was used.

For chromatography, as the silica gel, Silica Gel60 (70-230 mesh ASTM) from Merck Corporation or PSQ60B from Fuji Silysia Chemical Ltd. was used, or a pre-packed column {column: Hi-Flash™ Column (Silicagel) from Yamazen Corporation, size: any of S (16 × 60 mm), M (20 × 75 mm), L (26 × 100 mm), 2L (26 × 150 mm), and 3L (46 × 130 mm); or Biotage™ SNAP Ultra Silica Cartridge from Biotage Corporation, size: any of 10 g, 25 g, and 50 g} was used.

As the NH silica gel, CHROMATOREX NH-DM2035 from Fuji Silysia Chemical Ltd. was used, or a pre-packed column (column: Hi-Flash™ Column (Amino) from Yamazen Corporation, size: any of S (16 × 60 mm), M (20 × 75 mm), L (26 × 100 mm), 2L (26 × 150 mm), and 3L(46 × 130 mm); or Presep™ (Luer Lock) $NH_2$ (HC) from Wako Pure Chemical Industries, Ltd., size: any of type M (14 g /25 mL), type L (34 g/70 mL), type 2L (50 g/100 mL), and type 3L(110 g/200 mL)} was used.

As neutral alumina, Aluminium oxide 90 active neutral, 70-230 mesh, Merck, E6NXX was used.

As the names of the compounds shown below, those displayed on the "E-Notebook" Version 12 (PerkinElmer Co., Ltd.) were used, except for commonly used reagents.

Production Example 1

Synthesis of benzyl (3-(3-(benzyloxy)pryridin-2-yl)-4-methoxy-2-oxobut-3-en-1-yl)carbonate (E/Z mixture)

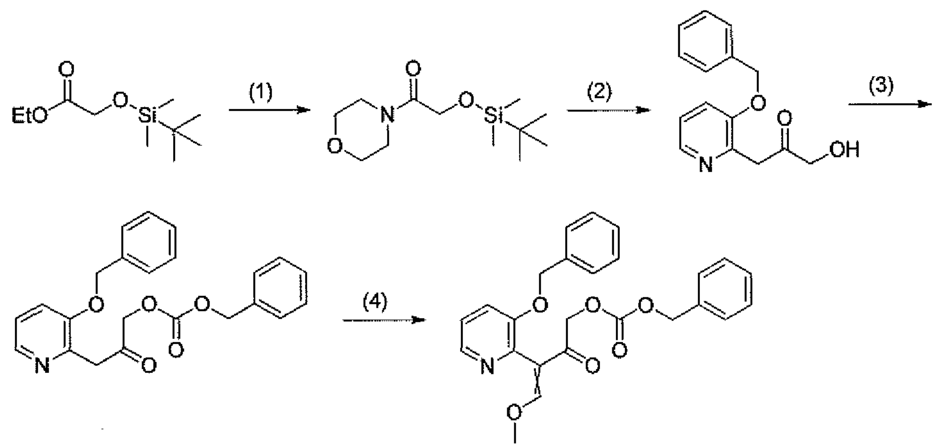


(1) Synthesis of 2-((t-butyldimethylsilyl)oxy)-1-morpholinoethanone

A mixture of 2,3,4,6,7,8-hexahydro-1H-pyriniido[1,2-a]pyrimidine (1.91 g, 13.7 mmol, 0.3 equivalents), ethyl 2-((t-butyldimethylsilyl)oxy)acetate (Journal of the American Chemical Society, 2011, 133(35), 14082-14089, Supporting

Information p.27) (CAS No. 67226-78-2) (10 g, 45.8 mmol, 1 equivalent), morpholine (4.41 mL, 50.4 mmol, 1.1 equivalents) and 2-methyltetrahydrofuran (50 mL) was stirred at 60°C overnight. The reaction mixture was allowed to return to room temperature, and then washed sequentially with a 5% aqueous ammonium chloride solution and water. The solution was concentrated under reduced pressure to afford the title compound (10.4 g).
$^1$H-NMR (400 MHz, $CDCl_3$) $\delta$ (ppm): 0.11 (s, 6H), 0.90 (s, 9H), 3.54-3.74 (m, 8H), 4.29 (s, 2H).
MS $[M+H]^+$ = 260

(2) Synthesis of 1-(3-(benzyloxy)pyridin-2-yl)-3-hydroxypropan-2-one

To a solution of 3-(benzyloxy)-2-methylpyridine (Journal of Medicinal Chemistry, 2008, 51(15), 4708-4714, Supporting Information, p.22) (CAS No. 177559-01-2) (4.4 g, 22.1 mmol, 1 equivalent) in 2-methyltetrahydrofuran (44 mL) was added n-butyllithium (2.65 M solution in n-hexane, 10.0 mL, 26.5 mmol, 1.2 equivalents) at -78°C. The reaction mixture was stirred at -78°C for 1 hour. The reaction mixture was added dropwise to a solution of 2-((t-butyldimethylsilyl)oxy)-1-morphohnoethanone (6.87 g, 26.5 mmol, 1.2 equivalents) in 2-methyltetrahydrofuran (13.2 mL) at -78°C. The reaction mixture was stirred at -78°C for 30 minutes. To the reaction mixture was added a 5% aqueous ammonium chloride solution, and the mixture was allowed to warm up to room temperature. The organic layer was separated. The resulting organic layer was washed with a 5% aqueous ammonium chloride solution. To the resulting organic layer was added 2 N hydrochloric acid (26.4 mL, 52.8 mmol, 2.39 equivalents), and the mixture was stirred at room temperature for 2 hours. The aqueous layer was separated. The resulting aqueous layer was neutralized with a 2 N aqueous sodium hydroxide solution, and extracted with ethyl acetate. The organic layer was dried over sodium sulfate and filtered, and then the resulting solution was concentrated under reduced pressure at 60°C to approximately five times the volume of 3-(benzyloxy)-2-methylpyridine used. The remaining solution was cooled down to room temperature. To this mixture was added n-heptane (50 mL). The precipitate was collected by filtration. The resulting solid was washed with MTBE to afford the title compound (3.53 g).
$^1$H-NMR (400 MHz, $CDCl_3$) $\delta$ (ppm): 3.48 (br. s, 1H), 4.03 (s, 2H), 4.28 (s, 2H), 5.08 (s, 2H), 7.12-7.25 (m, 2H), 7.30-7.55 (m, 5H), 8.16 (dd, J = 4.7, 1.6 Hz, 1H).
MS $[M+H]^+$ = 258

(3) Synthesis of benzyl ('3-(3-(benzyloxy)pyridin-2-yl)-2-oxopropyl)carbonate

To a solution of 1-(3-(benzyloxy)pyridin-2-yl)-3-hydroxypropan-2-one (21 g, 81.6 mmol, 1 equivalent) in THF (210 mL) were added pyridine (8.58 mL, 106 mmol, 1.3 equivalents) and 1-carbobenzoxy-3-methylimidazolium triflate (CAS No. 163080-99-7) (35.9 g, 97.9 mmol, 1.2 equivalents) at 0°C. The reaction mixture was stirred at room temperature for 16 hours, then MTBE, acetic acid (8.5 mL) and water were added, and the organic layer was separated. The organic layer was washed sequentially with water and a saturated aqueous sodium chloride solution. The organic layer was dried over anhydrous sodium sulfate, filtered, and then concentrated under reduced pressure to afford the title compound (33 g) as a crude product. This crude product was used for the next reaction without further purification.
MS $[M+H]^+$ = 392

(4) Synthesis of benzyl (3-(3-(benzyloxy)pyridin-2-yl)-4-methoxy-2-oxobut-3-en-1-yl)carbonate (E/Zmixture)

To a mixture of benzyl (3-(3-(benzyloxy)pyridin-2-yl)-2-oxopropyl)carbonate (33 g) and trimethyl orthoformate (CAS No. 149-73-5) (66 mL, 603 mmol) were added acetic anhydride (132 mL, 1.40 mol) and acetic acid (66 mL, 1.15 mol) at room temperature. The reaction mixture was stirred at 70°C for 3 hours. The reaction mixture was concentrated under reduced pressure. The residue was purified with silica gel column chromatography (silica gel, 15%-100% ethyl acetate/n-heptane) to afford the title compound (29.2 g).
$^1$H-NMR (400 MHz, $CDCl_3$) $\delta$ (ppm): 3.84 (s, 0.85 x 3H), 3.94 (s, 0.15 x 3H), 4.74 (s, 0.85 x 2H), 5.04 (s, 0.15 x 2H), 5.10 (s, 0.85 x 2H), 5.11 (s, 0.15 x 2H), 5.17 (s, 0.85 x 2H), 5.19 (s, 0.15 x 2H), 7.10-7.43 (m, 0.85 x 12H and 0.15 x 13H), 7.64 (s, 0.85 x 1H), 8.15 (dd, J = 4.0, 2.2 Hz, 0.15 x1H), 8.27 (dd, J = 4.8,1.5 Hz, 0.85 x 1H).
MS $[M+H]^+$ = 434

Referential Example 1

Synthesis of 2-fluoro-6-(3-fluoro-8-oxo-7-(pyridin-3-yl)-7,8-dihydro-6H-pyrano[3,2-b:5,4-b']dipyridin-9-yl)benzonitrile

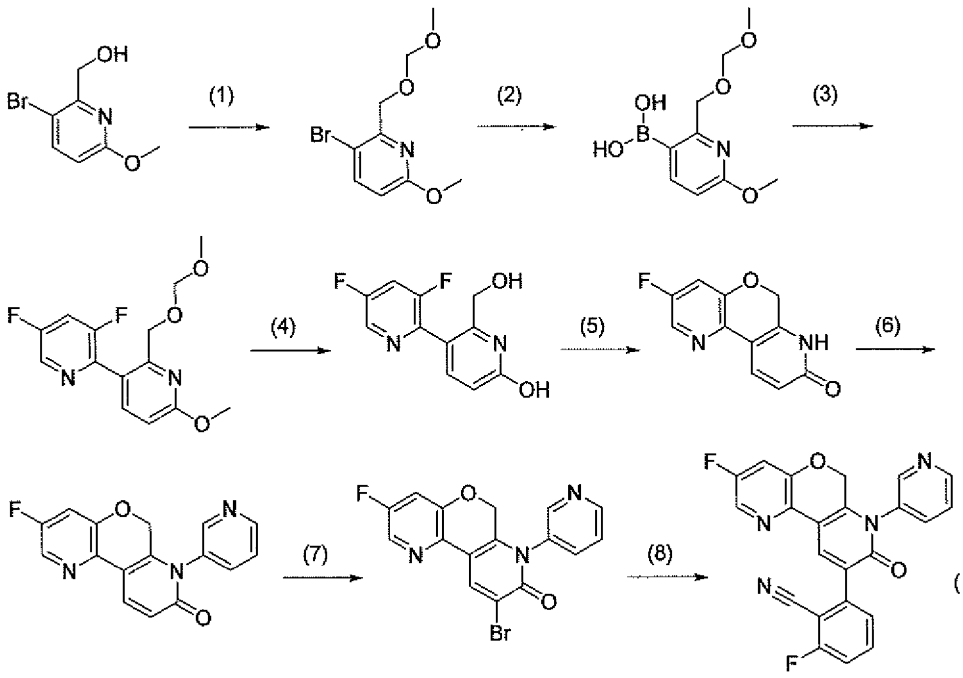


(I)

(1) Synthesis of 3-bromo-6-methoxy-2-((methoxymethoxy)methyl)pyridine

A mixture of (3-bromo-6-methoxypyridin-2-yl)methanol (CAS No. 623942-84-7) (2.15 g, 9.86 mmol, 1 equivalent), chloromethyl methyl ether (2.25 mL, 29.6 mmol, 3 equivalents) and N,N-diisopropylethylamine (8.61 mL, 49.3 mmol, 5 equivalents) was stirred in DCM (45 mL) at room temperature for 15 hours. The reaction mixture was concentrated under reduced pressure. The residue was purified with silica gel column chromatography (NH silica gel, 0%-5% ethyl acetate/n-heptane) to afford the title compound (2.22 g).
MS $[M+H]^+$ = 262

(2) Synthesis of (6-methoxy-2-((methoxymethoxy)methyl)pyridin-3-yl)boronic acid

A solution of 3-bromo-6-methoxy-2-((methoxymethoxy)methyl)pyridine (1.09 g, 4.16 mmol, 1 equivalent) in THF (20 mL) was cooled to -78°C, and n-butyllithium (2.69 M solution in n-hexane, 1.70 mL, 4.58 mmol, 1.1 equivalents) was added. The reaction mixture was stirred at the same temperature for 1 hour, and then trimethyl borate (0.696 mL, 6.24 mmol, 1.5 equivalents) was added. The reaction mixture was stirred for 12 hours while warming up to room temperature, and then concentrated under reduced pressure. To the residue was added a saturated aqueous ammonium chloride solution, and the mixture was extracted with DCM. The organic layer was concentrated under reduced pressure, and the residue was purified with silica gel column chromatography (silica gel, 10%-100% ethyl acetate/n-heptane) to afford the title compound (570 mg).
$^1$H-NMR (400 MHz, $CDCl_3$) $\delta$ (ppm): 3.42 (s, 3H), 3.95 (s, 3H), 4.75 (s, 2H), 4.79 (s, 2H), 6.32 (s, 2H), 6.71 (d, J = 8.2 Hz, 1H), 8.08 (d, J = 8.4 Hz, 1H).
MS $[M+H]^+$ = 228

(3) Synthesis of 3,5-difluoro-6'-methoxy-2'-((methoxymethoxy)methyl)-2,3'-bipyridine

A mixture of (6-methoxy-2-((methoxymethoxy)methyl)pyridin-3-yl)boronic acid (570 mg, 2.51 mmol, 1 equivalent), 2-bromo-3,5-difluoropyridine (CAS No. 660425-16-1) (560 mg, 2.89 mmol, 1.15 equivalents), potassium fluoride (438 mg, 7.53 mmol, 3 equivalents), $(Ataphos)_2PdCl_2$ (89 mg, 0.126 mmol, 0.05 equivalents), 1,4-dioxane (12 mL) and water (3 mL) was reacted in a microwave reactor at 130°C for 4 hours. The reaction mixture was cooled down to room temperature, and then directly purified with silica gel column chromatography (silica gel on NH silica gel, 5%-35% ethyl acetate/n-heptane) to afford the title compound (648 mg).
MS $[M+H]^+$ = 297

(4) Synthesis of 3,5-difluoro-2'-(hydroxymethyl)-[2,3'-bipyridin]-6'-ol

A mixture of 3,5-difluoro-6'-methoxy-2'-((memoxymethoxy)methyl)-2,3'-bipyridine (648 mg, 2.19 mmol, 1 equivalent) and a 48% aqueous hydrobromic acid solution (1.98 mL, 17.5 mmol, 8 equivalents) was stirred in THF (15 mL) at 55°C for 8 hours. The reaction mixture was cooled down to room temperature, and then concentrated under reduced pressure. To the residue were added a saturated aqueous sodium hydrogen carbonate solution and DCM. The mixture was stirred at room temperature for 1 hour, and then the resulting precipitate was collected by filtration. The resulting solid was washed with water to afford the title compound (349 mg).
$^1$H-NMR (400 MHz, DMSO-$d_6$) δ (ppm): 4.33 (s, 2H), 6.35 (d, J = 9.3 Hz, 1H), 7.51 (dd, J = 9.3,1.8 Hz, 1H), 8.04 (ddd, J= 10.1,8.9,2.5 Hz, 1H), 8.57 (d, J = 2.4 Hz, 1H).
MS $[M+H]^+$ = 239

(5) Synthesis of 3-fluoro-6H-pyranol[3,2-b:5,4-b']dipyridin-8(7H)-one

A mixture of 3,5-difluoro-2'-(hydroxymethyl)-[2,3'-bipyridin]-6'-ol (329 mg, 1.38 mmol, 1 equivalent) and potassium carbonate (573 mg, 4.14 mmol, 3 equivalents) was stirred in DMF (7 mL) at 100°C for 5 hours. The reaction mixture was cooled down to room temperature, and then an aqueous ammonium chloride solution was added. The mixture was stirred at room temperature for 30 minutes, and then the resulting precipitate was collected by filtration. The resulting solid was washed with water and n-heptane to afford the title compound (251 mg).
$^1$H-NMR (400 MHz, DMSO-$d_6$) δ (ppm): 5.20 (s, 2H), 6.46 (d, J = 8.8 Hz, 1H), 7.38 (dd, J = 9.7,2.4 Hz, 1H), 8.04 (d, J = 9.3 Hz, 1H), 8.19 (d, J = 2.6 Hz, 1H).
MS $[M+H]^+$ = 219

(6) Synthesis of 3-fluoro-7-(pyridin-3-yl)-6H-pyrano[3,2-b:5,4-b']dipyridin-8(7H)-one

A mixture of 3-fluoro-6H-pyrano[3,2-b:5,4-b']dipyridin-8(7H)-one (250 mg, 1.15 mmol, 1 equivalent), silver carbonate (474 mg, 1.72 mmol, 1.5 equivalents), copper(I) iodide (131 mg, 0.687 mmol, 0.6 equivalents) and pyridine (0.927 mL, 11.5 mmol, 10 equivalents) was stirred in a mixed solvent of DMF (7 mL) and DMSO (9 mL) at 80°C for 20 minutes. To the reaction mixture was slowly added a mixed solution of pyridine-3-boronic acid 1,3-propanediol cyclic ester (CAS No. 131534-65-1) (373 mg, 2.29 mmol, 2 equivalents) in DMF (4 mL) and DMSO (1 mL). The reaction mixture was stirred at 80°C for 20 hours, then applied to silica gel pad (NH silica gel and silica gel) and eluted with ethyl acetate. The resulting solution was concentrated under reduced pressure. To the residue was added water, and the mixture was extracted with ethyl acetate. The organic layer was washed with a saturated aqueous sodium chloride solution, and then concentrated under reduced pressure. The residue was purified with silica gel column chromatography (NH silica gel on silica gel, 10%-100% ethyl acetate/n-heptane, 10% methanol/ethyl acetate) to afford the title compound (43.5 mg).
$^1$H-NMR (400 MHz, $CDCl_3$) δ (ppm): 4.644.73 (m, 1H), 4.77-4.86 (m, 1H), 6.78 (d, J = 9.5 Hz, 1H), 6.94 (dd, J = 8.9, 2.5 Hz, 1H), 7.51-7.58 (m, 1H), 7.65-7.72 (m, 1H), 8.14 (d, J = 2.6 Hz, 1H), 8.29 (d, J = 9.7 Hz, 1H), 8.53 (d, J= 2.6 Hz, 1H), 8.79 (dd, J = 4.8, 1.5 Hz, 1H).
MS $[M+H]^+$ = 296

(7) Synthesis of 9-bromo-3-fluoro-7-(pyridin-3-yl)-6H-pyrano[3,2-b:5,4-b']dipyridin-8(7H)-one

Amixture of 3-fluoro-7-(pyridin-3-yl)-6H-pyrano[3,2-b:5,4-b']dipyridin-8(7H)-one (43.5 mg, 0.147 mmol, 1 equivalent) and NBS (31.5 mg, 0.177 mmol, 1.2 equivalents) was stirred in acetonitrile (3 mL) at room temperature for 15 hours. The reaction mixture was directly purified with silica gel column chromatography (NH silica gel on silica gel, 10%-100% ethyl acetate/n-heptane) to afford the title compound (33 mg).
$^1$H-NMR (400 MHz, $CDCl_3$) δ (ppm): 4.60-4.70 (m, 1H), 4.74-4.83 (m, 1H), 6.95 (dd, J = 8.6,2.0 Hz, 1H), 7.56 (dd, J = 8.1, 4.8 Hz, 1H), 7.65-7.72 (m, 1H), 8.14 (d, J = 2.4 Hz, 1H), 8.53 (d, J = 2.4 Hz, 1H), 8.71 (s, 1H), 8.81 (dd, J = 4.9,1.6 Hz, 1H).
MS $[M+Na]^+$ = 396

(8) Synthesis of 2-fluoro-6-(3-fluoro)-8-oxo-7-(pyridin-3-yl)-7,8-dihydro-6H-pyrano[3,2-b:5,4-b']dipyridin-9-yl)benzonitrile

A mixture of 9-bromo-3-fluoro-7-(pyridin-3-yl)-6H-pyrano[3,2-b:5,4-b']dipyridin-8(7H)-one (16.5 mg, 0.044 mmol, 1 equivalent), 2-cyano-3-fluorophenylboronic acid pinacol ester (CAS No. 765916-91-4) (14.2 mg, 0.057 mmol, 1.3 equivalents), $(Ataphos)_2PdCl_2$ (1.56 mg, 2.21 μmol, 0.05 equivalents), triethylamine (0.025 mL, 0.176 mmol, 4 equivalents), 1,4-dioxane (0.5 mL) and water (0.15 mL) was reacted in a microwave reactor at 130°C for 2.5 hours. The

reaction solution was directly purified with silica gel column chromatography (NH silica gel on silica gel, 5%-90% ethyl acetate/n-heptane) to afford the title compound (11.0 mg).

$^1$H-NMR (400 MHz, $CDCl_3$) $\delta$ (ppm): 4.71-4.80 (m, 1H), 4.864.95 (m, 1H), 6.97 (dd, J = 8.8, 2.4 Hz, 1H), 7.20-7.28 (m, 1H), 7.46 (d, J = 7.7 Hz, 1H), 7.57 (dd, J = 8.2, 4.8 Hz, 1H), 7.63 (td, J = 8.2, 5.8 Hz, 1H), 7.77 (ddd, J = 8.1, 2.6,1.6 Hz, 1H), 8.14 (d, J = 2.6 Hz, 1H), 8.51 (s, 1H), 8.60 (d, J = 2.6 Hz, 1H), 8.81 (dd, J= 4.9, 1.6 Hz, 1H).

MS $[M+H]^+$ = 415

Referential Example 2

Synthesis of 2-fluoro-6-(8-oxo-7-(pyrimidin-5-yl)-7,8-dihydro-6H-pyrano[3,2-b:5,4-b']dipyridin-9-yl)benzonitrile

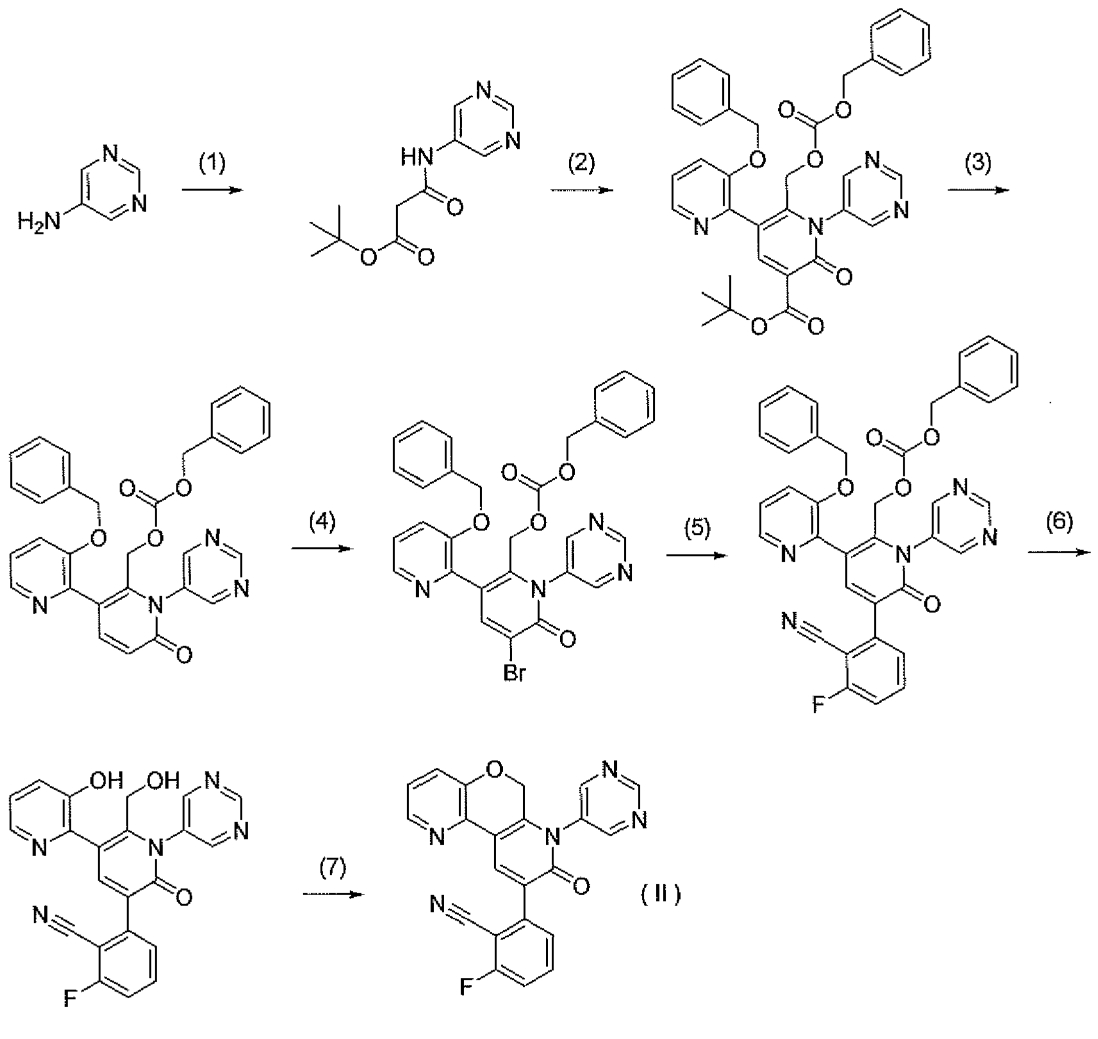


(II)

(1) Synthesis of t-butyl 3-oxo-3-(pyrimidin-5-ylamino)propanoate

To a mixture of 5-aminopyrimidine (CAS No. 591-55-9) (2.00 g, 21.0 mmol, 1 equivalent), triethylamine (14.7 mL, 105 mmol, 5 equivalents), EDC (7.26 g, 37.9 mmol, 1.8 equivalents), N,N-dimethyl-4-aminopyridine (0.257 g, 2.10 mmol, 0.1 equivalents) and DCM (60.0 mL) was added dropwise 3-(t-butoxy)-3-oxopropionic acid (CAS No. 40052-13-9) (4.86 mL, 31.5 mmol, 1.5 equivalents), and the mixture was stirred at room temperature for 3 days. To the reaction mixture were added water and ethyl acetate. The organic layer was washed with water and a saturated aqueous sodium chloride solution. The organic layer was dried over magnesium sulfate, filtered, and then concentrated under reduced pressure. The residue was purified with silica gel column chromatography (silica gel, 25%-71% ethyl acetate/n-heptane) to afford the title compound (4.73 g).

$^1$H-NMR (400 MHz, $CDCl_3$) $\delta$ (ppm): 1.52 (s, 9H), 3.44 (s, 2H), 8.98 (s, 1H), 9.03 (s, 2H), 9.77 (br. s, 1H).

(2) Synthesis of t-butyl 3-(benzyloxy)-2'-((((benzyloxy)carbonyl)oxy)methyl)-6'-oxo-1'-(pyrimidin-5-yl)-1',6'-dihydro-[2,3'-bipyridine]-5'-carboxylate

To a solution of benzyl (3-(3-(benzyloxy)pyridin-2-yl)-4-methoxy-2-oxobut-3-en-1-yl)carbonate (E/Z mixture) obtained in Production Example 1 (3.05 g, 7.03 mmol, 1 equivalent) in propionitrile (50.0 mL) were sequentially added t-butyl 3-oxo-3-(pyrimidin-5-ylamino)propanoate (2.00 g, 8.43 mmol, 1.2 equivalents), lithium bromide (1.22 g, 14.1 mmol, 2 equivalents) and triethylamine (3.43 mL, 24.6 mmol, 3.5 equivalents) at 0°C. After stirring at the same temperature for 5 minutes, the reaction mixture was warmed up to room temperature and stirred for 12 hours. To the reaction mixture was added water, and the mixture was extracted with ethyl acetate. The combined organic layers were washed with a saturated aqueous sodium chloride solution. The organic layers were dried over sodium sulfate, filtered, and then concentrated under reduced pressure. The residue was purified with silica gel column chromatography (NH silica gel, 20%-75% ethyl acetate/n-heptane). The resulting crude product was triturated with a mixed solution of MTBE/n-heptane to afford the title compound (2.30 g).
$^1$H-NMR (400 MHz, $CDCl_3$) $\delta$ (ppm): 1.55 (s, 9H), 4.70 (s, 2H), 4.93 (s, 2H), 5.07 (s, 2H), 7.24-7.27 (m, 1H), 7.29-7.42 (m, 11H), 8.26 (dd, J = 4.7,1.6 Hz, 1H), 8.34 (s, 1H), 8.62 (d, J = 0.8 Hz, 2H), 9.21 (s, 1H).

(3) Synthesis of benzyl ((3-(benzyloxy)-6'-oxo-1'-(pyrimidin-5-yl)-1',6'-dihydro-[2,3'-bipyridin]-2'-yl)methyl)carbonate

To t-butyl 3-(benzyloxy)-2'-((((benzyloxy)carbonyl)oxy)methyl)-6'-oxo-1'-(pyrimidin-5-yl)-1',6'-dihydro-[2,3'-bipyridine]-5'-carboxylate (1.56 g, 2.51 mmol, 1 equivalent) was added hydrogen chloride (4 M solution in ethyl acetate) (6.28 mL, 25.1 mmol, 10 equivalents), and the mixture was stirred at room temperature for 30 minutes. The reaction mixture was concentrated under reduced pressure, and to the resulting residue were added DMSO (46.8 mL) and lithium acetate dihydrate (2.56 g, 25.1 mmol, 10 equivalents), and the reaction mixture was stirred at 120°C for 4 hours. The reaction mixture was cooled to room temperature, and then to the reaction mixture was added water, and the mixture was extracted with ethyl acetate. The combined organic layers were washed with water and a saturated aqueous sodium chloride solution. The organic layers were dried over magnesium sulfate, filtered, and then concentrated under reduced pressure. The resulting residue was purified with silica gel column chromatography (silica gel, 50%-83% ethyl acetate/n-heptane, 5% methanol/ethyl acetate) to afford the title compound (588 mg).
$^1$H-NMR (400 MHz, $CDCl_3$) $\delta$ (ppm): 4.69 (s, 2H), 4.94 (s, 2H), 5.08 (s, 2H), 6.75 (d, J = 9.8 Hz, 1H), 7.27-7.42 (m, 12H), 7.60 (d, J = 9.4 Hz, 1H), 8.25 (dd, J = 4.7, 1.6 Hz, 1H), 8.67 (s, 2H), 9.23 (s, 1H).
MS $[M+H]^+$ = 521

(4) Synthesis of benzyl ((3-(benzyloxy)-5'-bromo-6'-oxo-1'-(pyrimidin-5-yl)-1',6'-dihydro-[2,3'-bipyridin]-2'-yl)methyl)carbonate

To a solution of benzyl ((3-(benzyloxy)-6'-oxo-1'-(pyrimidin-5-yl)-1',6'-dihydro-[2,3'-bipyridin]-2'-yl)methyl)carbonate (560 mg, 1.08 mmol, 1 equivalent) in DMF (5.00 mL) was added NBS (287 mg, 1.61 mmol, 1.5 equivalents) at room temperature, and the mixture was stirred at room temperature for 18 hours. To the reaction mixture was added water, and the mixture was extracted with ethyl acetate. The combined organic layers were washed with water and a saturated aqueous sodium chloride solution. The organic layers were dried over magnesium sulfate, filtered, and then concentrated under reduced pressure. The resulting residue was purified with silica gel column chromatography (silica gel, 50%-77% ethyl acetate/n-heptane) to afford the title compound (595 mg).
$^1$H-NMR (400 MHz, $CDCl_3$) $\delta$ (ppm): 4.66 (s, 2H), 4.93 (s, 2H), 5.08 (s, 2H), 7.26-7.42 (m, 12H), 8.03 (s, 1H), 8.25 (dd, J = 4.5,1.4 Hz, 1H), 8.64 (s, 2H), 9.23 (s, 1H).
MS $[M+H]^+$ = 599,601

(5) Synthesis of benzyl ((3-(benzyloxy)-5'-(2-cyano-3-fluorophenyl)-6'-oxo-1'-(pyrimidin-5-yl)-1',6'-dihydro-[2,3'-bipyridin]-2'-yl)methyl)carbonate

A mixture of benzyl ((3-(benzyloxy)-5'-bromo-6'-oxo-1'-(pyrimidin-5-yl)-1',6'-dihydro-[2,3'-bipyridin]-2'-yl)methyl)carbonate (590 mg, 0.984 mmol, 1 equivalent), 2-cyano-3-fluorophenylboronic acid pinacol ester (CAS No. 765916-91-4) (365 mg, 1.48 mmol, 1.5 equivalents), $(Ataphos)_2PdCl_2$ (34.8 mg, 0.049 mmol, 0.05 equivalents), potassium fluoride (172 mg, 2.95 mmol, 3 equivalents), 1,4-dioxane (15.0 mL) and water (3.0 mL) was reacted in a microwave reactor at 120°C for 30 minutes. To the reaction mixture was added water, and the mixture was extracted with ethyl acetate. The combined organic layers were washed with water and a saturated aqueous sodium chloride solution. The organic layers were dried over magnesium sulfate, filtered, and then concentrated under reduced pressure. The resulting residue was purified with silica gel column chromatography (silica gel, 50%-83% ethyl acetate/n-heptane) to afford the title compound (511 mg).

$^1$H-NMR (400 MHz, $CDCl_3$) δ (ppm): 4.76 (s, 2H), 4.96 (s, 2H), 5.13 (s, 2H), 7.21 (t, J = 8.6 Hz, 1H), 7.27-7.37 (m, 13H), 7.57 (td, J = 8.2, 5.9 Hz, 1H), 7.83 (s, 1H), 8.23 (dd, J = 4.7,1.2 Hz, 1H), 8.73 (s, 2H), 9.24 (s, 1H).
MS $[M+H]^+$ = 640

(6) Synthesis of 2-fluoro-6-(3-hydroxy-2'-(hydroxymethyl)-6'-oxo-1'-(pyrimidin-5-yl)-1',6'-dihydro-[2,3'-bipyridin]-5'-yl)benzonitrile

A mixture of benzyl ((3-(benzyloxy)-5'-(2-cyano-3-fluorophenyl)-6'-oxo-1'-(pyrimidin-5-yl)-1',6'-dihydro-[2,3'-bipyridin]-2'-yl)methyl)carbonate (505 mg, 0.79 mmol, 1 equivalent), 5% palladium on carbon (84.0 mg, 0.039 mmol, 0.05 equivalents) and ethanol (20.0 mL) was stirred vigorously at room temperature under a hydrogen atmosphere for 6 hours. The reaction mixture was purged with nitrogen, and then filtered. The resulting filtrate was concentrated under reduced pressure to afford the title compound (311 mg).
MS $[M+H]^+$ = 416

(7) Synthesis of 2-fluoro-6-(8-oxo-7-(pyrimidin-5-yl)-7,8-dihydro-6H-pyrano[3,2-b:5,4-b']dipyridin-9-yl)benzonitrile

To a mixture of 2-fluoro-6-(3-hydroxy-2'-(hydroxymethyl)-6'-oxo-1'-(pyrimidin-5-yl)-1',6'-dihydro-[2,3'-bipyridin]-5'-yl)benzonitrile (311 mg, 0.749 mmol, 1 equivalent), triphenylphosphine (295 mg, 1.12 mmol, 1.5 equivalents) and THF (10.0 mL) was slowly added DMEAD (263 mg, 1.12 mmol, 1.5 equivalents) at room temperature, and the mixture was stirred for 10 minutes. To the reaction mixture was added water, and the mixture was extracted with ethyl acetate. The combined organic layers were washed with water and a saturated aqueous sodium chloride solution. The organic layers were dried over magnesium sulfate, filtered, and then concentrated under reduced pressure. The resulting residue was purified with silica gel column chromatography (silica gel, 25%-63% ethyl acetate/n-heptane) to afford the title compound (110 mg).
$^1$H-NMR (400 MHz, $CDCl_3$) δ (ppm): 4.82 (s, 2H), 7.12-7.17 (m, 1H), 7.19-7.23 (m, 1H), 7.24-7.26 (m, 1H), 7.44 (d, J = 7.8 Hz, 1H), 7.65 (td, J = 8.1, 5.7 Hz, 1H), 8.27 (dd, J = 4.5, 1.4 Hz, 1H), 8.59 (s, 1H), 8.81 (s, 2H), 9.39 (s, 1H).
MS $[M+H]^+$ = 398

Referential Example 3

Synthesis of 2-fluoro-6-(7-(2-methylpyrimidin-5-yl)-8-oxo-7,8-dihydro-6H-pyrano[3,2-b:5,4-b']dipyridin-9-yl)benzonitrile

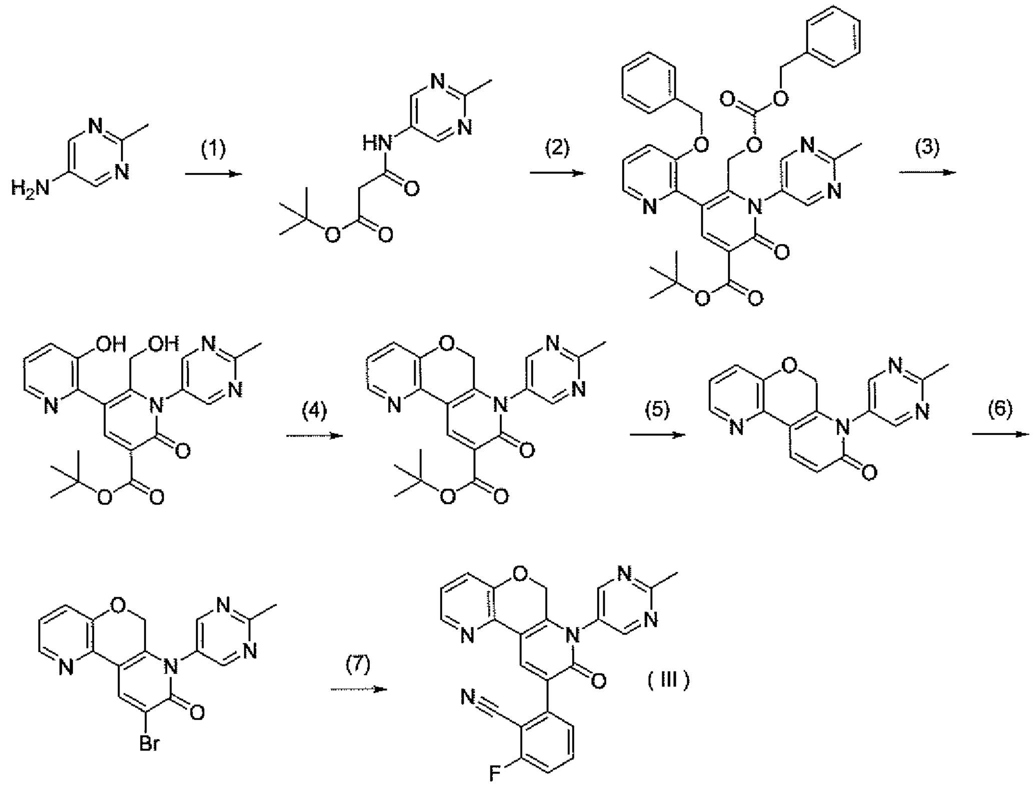


( III )

(1) Synthesis of t-butyl 3-((2-methylpyrimidin-5-yl)amino)-3-oxopropanoate

A mixture of 3-(t-butoxy)-3-oxopropionic acid (CAS No. 40052-13-9) (1.61 g, 10.1 mmol, 1.1 equivalents), 2-methyl-5-pyrimidinamine (CAS No. 39889-94-6) (1.00 g, 9.16 mmol, 1.0 equivalent), triethylamine (1.53 mL, 11.0 mmol, 1.2 equivalents), EDC (2.11 g, 11.0 mmol, 1.2 equivalents) and DCM (20 mL) was heated to reflux for 1 hour. The reaction mixture was cooled down to room temperature, then a saturated aqueous sodium hydrogen carbonate solution was added, and the mixture was extracted with DCM. The organic layer was concentrated under reduced pressure. The residue was purified with silica gel column chromatography (silica gel, 50%-100% ethyl acetate/n-heptane) to afford the title compound (2.21 g).
$^1$H-NMR (400 MHz, $CDCl_3$) $\delta$ (ppm): 1.53 (s, 9H), 2.72 (s, 3H), 3.43 (s, 2H), 8.91 (s, 2H), 9.64 (br. s, 1H).

(2) Synthesis of t-butyl 3-(benzyloxy)-2'-((((benzyloxy)carbonyl)oxy)methyl)-1'-(2-methylpyrimidin-5-yl)-6'-oxo-1',6'-dihydro-[2,3'-bipyridine]-5'-carboxylate

A mixture of t-butyl 3-((2-methylpyrimidin-5-yl)amino)-3-oxopropanoate (313 mg, 1.25 mmol, 1.2 equivalents), lithium bromide (180 mg, 2.08 mmol, 2.0 equivalents), benzyl (3-(3-(benzyloxy)pyridin-2-yl)4-methoxy-2-oxobut-3-en-1-yl)carbonate (E/Z mixture) obtained in Production Example 1 (450 mg, 1.04 mmol, 1.0 equivalents), triethylamine (0.506 mL, 3.63 mmol, 3.5 equivalents) and propionitrile (5.00 mL) was stirred at room temperature for 10 minutes. The reaction mixture was directly purified with silica gel column chromatography (silica gel, 40%-100% ethyl acetate/n-heptane) to afford the title compound (480 mg).
MSm/z = 636

(3) Synthesis of t-butyl 3-hydroxy-2'-(hydroxymethyl)-1'-(2-methylpyrimidin-5-yl)-6'-oxo-1',6'-dihydro-[2,3'-bipyridine]-5'-carboxylate

A mixture of t-butyl 3-(benzyloxy)-2'-((((benzyloxy)carbonyl)oxy)methyl)-1'-(2-methylpyrimidin-5-yl)-6'-oxo-1',6'-dihydro-[2,3'-bipyridine]-5'-carboxylate (480 mg, 0.756 mmol, 1.0 equivalents), 10% palladium on carbon (water content, 53.9%) (80.0 mg, 0.035 mmol, 0.046 equivalents) and methanol (3.00 mL) was stirred at room temperature under a hydrogen atmosphere for 40 minutes. The reaction mixture was filtered through Celite™, and the residue was washed with ethyl acetate. The resulting filtrate was concentrated under reduced pressure. The residue was purified with silica gel column chromatography (silica gel, 0%-20% methanol/ethyl acetate) to afford the title compound (250 mg).

MS $[M+H]^+$ = 411

(4) Synthesis of t-butyl 7-(2-methylpyrimidin-5-yl)-8-oxo-7,8-dihydro-6H-pyrano[3,2-b:5,4-b']dipyridine-9-carboxylate

To a mixture of t-butyl 3-hydroxy-2'-(hydroxymethyl)-1'-(2-methylpyrimidin-5-yl)-6'-oxo-1',6'-dihydro-[2,3'-bipyridine]-5'-carboxylate (250 mg, 0.609 mmol, 1.0 equivalents), triphenylphosphine (224 mg, 0.853 mmol, 1.4 equivalents) and THF (3.00 mL) was added a solution of DMEAD (200 mg, 0.853 mmol, 1.4 equivalents) in THF (1.00 mL) at 0°C. After the progress of the reaction was confirmed, the reaction mixture was concentrated under reduced pressure to about one half of the original solution volume. The residue was purified with silica gel column chromatography (silica gel, 40%-100% ethyl acetate/n-heptane) to afford the title compound (239 mg).
MS $[M+H]^+$ = 393

(5) Synthesis of 7-(2-methylpyrimidin-5-yl)-6H-pyrano[3,2-b:5,4-b']dipyridin-8(7H)one

To t-butyl 7-(2-methylpyrimidin-5-yl)-8-oxo-7,8-dihydro-6H-pyrano[3,2-b:5,4-b']dipyridine-9-carboxylate (239 mg, 0.609 mmol, 1.0 equivalent) was added TFA (3.00 mL), and the reaction mixture was concentrated under reduced pressure. To the resulting residue were added DMSO (3.00 mL) and lithium acetate dihydrate (311 mg, 3.05 mmol, 5.0 equivalents), and the reaction mixture was stirred at 120°C for 30 minutes. To the reaction mixture was added a 10% aqueous sodium chloride solution, and the mixture was extracted with ethyl acetate. The organic layer was dried over magnesium sulfate, filtered, and then concentrated under reduced pressure. The resulting residue was purified with silica gel column chromatography (NH silica gel, 60%-80% ethyl acetate/n-heptane). The resulting crude product was purified again with silica gel column chromatography (silica gel, 0%-15% methanol/ethyl acetate) to afford the title compound (34.0 mg).
MS $[M+H]^+$ = 293

(6) Synthesis of 9-bromo-7-(2-methylpyrimidin-5-yl)-6H-pyrano[3,2-b:5,4-b']dipyridin-8(7H)-one

A mixture of 7-(2-methylpyrimidin-5-yl)-6H-pyrano[3,2-b:5,4-b']dipyridin-8(7H)-one (34.0 mg, 0.116 mmol, 1.0 equivalent), NBS (31.1 mg, 0.174 mmol, 1.5 equivalents) and DMF (3.00 mL) was stirred at 50°C for 30 minutes. The reaction mixture was concentrated under reduced pressure. The resulting residue was purified with silica gel column chromatography (silica gel, 0%-25% methanol/ethyl acetate) to afford the title compound (22.0 mg).
MS $[M+H]^+$ = 371

(7) Synthesis of 2-fluoro-6-(7-(2-methylpyrimidin-5-yl)-8-oxo-7,8-dihydro-6H-pyrano[3,2-b:5,4-b']dipyridin-9-yl)benzonitrile

A mixture of 9-bromo-7-(2-methylpyrimidin-5-yl)-6H-pyrano[3,2-b:5,4-b']dipyridin-8(7H)-one (22.0 mg, 0.059 mmol, 1.0 equivalent), 2-cyano-3-fluorophenylboronic acid pinacol ester (CAS No. 765916-91-4) (22.0 mg, 0.089 mmol, 1.5 equivalents), $(Ataphos)_2PdCl_2$ (4.20 mg, 5.93 μmol, 0.1 equivalents), potassium fluoride (10.3 mg, 0.178 mmol, 3.0 equivalents), 1,4-dioxane (0.8 mL) and water (0.4 mL) was reacted in a microwave reactor at 140°C for 10 minutes. The reaction mixture was directly purified with silica gel column chromatography (silica gel, 80%-100% ethyl acetate/n-heptane). The resulting crude product was purified again with silica gel column chromatography (NH silica gel, 50%-80% ethyl acetate/n-heptane) to afford the title compound (11.5 mg).
$^1$H-NMR (400 MHz, $CDCl_3$) δ (ppm): 2.88 (s, 3H), 4.84 (s, 2H), 7.08-7.32 (m, 3H), 7.44 (d, J = 7.7 Hz, 1H), 7.64 (td, J = 8.1, 5.5 Hz, 1H), 8.26 (dd, J = 4.8, 1.5 Hz, 1H), 8.58 (s, 1H), 8.68 (s, 2H).
MS $[M+H]^+$ = 412

(Pharmacological Test Example)

The present inventors conducted, or can conduct the following tests to confirm AMPA receptor inhibitory action, tremor-suppressing effect, SFN-suppressing effect, anti-dystonia effect, anticonvulsant effect against AD-related epilepsy, anticonvulsant effect against glioma-related epilepsy, and antitumor effect against glioma.

[Test Example 1] AMPA-induced intraneuronal calcium entry-suppressing action

With regard to the AMPA receptor inhibitory action of the compounds, AMPA-induced intraneuronal calcium entry-suppressing action was examined using a primary culture system of embryonic rat cerebral cortex neurons.
<Culture conditions> Whole fetuses were removed from the uteri of Wistar rats at embryonic day 18 (Charles

River Japan) anesthetized with isoflurane. Whole brains were removed from the whole fetuses, and the cerebral cortex was separated in Leibovitz's L-15 medium, or Hanks balanced salt solution containing 20% fetal bovine serum. The resulting material was treated with trypsin/DNase solution at 37°C for 45 to 60 minutes. After the serum was added to stop the trypsin reaction, the resulting material was centrifuged at 1200 to 1500 rpm for 3 to 5 minutes. After the supernatant was removed, Neurobasal medium (containing 2% B-27 supplement and the like; hereinafter "Neurobasal medium") was added, and the cells were uniformly dispersed by gently pipetting. The cell suspension was slightly stirred, and then filtered through a nylon mesh filter. Viable cells were counted using a hemocytometer, and diluted with Neurobasal medium to 8 to 10 × $10^5$ cells/mL. The cells were seeded into 96 well plates at 100 μL per well and cultured in a $CO_2$ incubator (5%$CO_2$, 37°C). The medium was replaced after 24 hours, and neurons cultured for 7 to 21 days were used.

On the day of the assay, the medium was removed and replaced with a calcium measurement solution containing 5 to 10 μmol/L of Fura 2-AM (140 mmol/L sodium chloride, 5 mmol/L potassium chloride, 2 mmol/L magnesium chloride, 3 mmol/L calcium chloride, 24 mmol/L D(+)- glucose, 10 mmol/L HEPES, 1 μmol/L MK-801, pH 7.4), and the cells were treated in a $CO_2$ incubator (5%$CO_2$, 37°C) for 1 to 2 hours. The calcium measurement solution containing the Fura-2 AM solution was subsequently removed, and the neurons in each well were washed twice with the calcium measurement solution for each well, and then 50 μL of the calcium measurement solution was added to each well. Subsequently, 50 μL of a test compound or medium prepared at a concentration 3-fold higher than the final concentration was added, and the cells were pre-treated for about 15 minutes. The plates were subsequently placed on the Fluorescence Drug Screening System 6000 (Hamamatsu Photonics K.K.). The cells were stimulated by adding to each well 50 μL of an AMPA stimulating solution prepared to 3.3 μmol/L with the calcium measurement solution. Changes in calcium concentration were measured as the changes in fluorescence intensity at excitation wavelengths of 340 nm and 380 nm (measurement wavelength: 540 nm). The rate of change of the intracellular calcium concentration was calculated in accordance with the following equation:

$$\text{Rate of increase of the intracellular calcium concentration (\% control)} = (T_{post} - T_{pre})/(C_{post} - C_{pre}) \times 100$$

wherein $T_{post}$: the proportion of fluorescence of sample wells after stimulation; $T_{pre}$: the proportion of fluorescence of sample wells before stimulation; $C_{post}$: the proportion of fluorescence of control wells after stimulation; and $C_{pre}$: the proportion of fluorescence of control wells before stimulation.

The $IC_{50}$ value of the test compound for AMPA inhibition was determined from the rates of increase of the intracellular calcium concentration at various concentrations.

The results are shown in Table 1. These results revealed that the compounds (I) to (III) have sufficient AMPA receptor inhibitory action.

[Table 1]

| Sample | $IC_{50}$ (nM) |
|---|---|
| Compound (I) | 19.40 |
| Compound (II) | 29.00 |
| Compound (III) | 44.26 |

[Test Example 2] Rat harmaline model

To confirm tremor-suppressing effect, a harmaline model test with rats was conducted. In this model, balance disorder observed after administration of harmaline serves as an evaluation index (Neuroscience Letter, 590, p84-90 (2015)).

<Method>

Four-week-old male SD rats (Charles River) were subjected to the test (two treatments in total, n = 5 in each treatment). Balance disorder (one of tremor symptoms) induced by harmaline was used as an evaluation index (the above literature).

The compound was prepared for oral administration in 0.4% methylcellulose/5% Cremophor/5% 1 N hydrochloric acid/10% dimethylsulfoxide solution to reach an administration volume of 5 mL/kg, and orally administered. As

a negative control, only the mixed solvent without the compound (Vehicle) was used. Harmaline (Sigma-Aldrich Co. LLC) was diluted with physiological saline (Otsuka Pharmaceutical Co., Ltd.) to prepare 4 mg/mL harmaline solution. Twenty minutes after administration of the compound, the 4 mg/mL harmaline solution was intraperitoneally administered (dose: 20 mg/kg). Balance disorder was observed during 10 minutes from 5 minutes to 15 minutes after the administration of harmaline, and scored in four grades of 0, 1, 2, and 3.

The results were represented as mean ± standard deviation. Dunnett's multiple comparison test was used for statistical analysis, and results with $p < 0.05$ were determined to be of statistically significant difference.

<Results>

The results of the harmaline model are shown in Table 2. Pre-administration of the compound (I) led to statistically significant reduction of tremor scores. This result indicates the efficacy of the drug of the present application to animal models of essential tremor.

[Table 2]

| Treatment | Tremor score |
|---|---|
| Vehicle | 2.2±0.3 |
| Compound (I) 1 mg/kg | 1.3±0.2 |
| Compound (I) 2 mg/kg | 0.6±0.3* |
| Compound (I) 4 mg/kg | 0.4±0.2* |
| *p<0.05 versus Vehicle (Dunnett's Test) | |

[Test Example 3] Rat STZ model

To confirm analgesic effect in an SFN model, an STZ model test with rats was conducted. The effect of the compound on pain induced 2 weeks after administration of STZ was examined.

<Method>

Seven-week-old male SD rats (Charles River) were used for the test. To STZ aliquoted in advance into shading bottles, ice-cooled physiological saline (Otsuka Pharmaceutical Factory, Inc.) was added to reach 65 mg/mL, and the resultant was gently inversion-mixed with ice-cooling to dissolve STZ. After dissolving, the resultant was stored under ice-cooling, and used within 20 minutes from the initiation of preparation. About 20 hours after fasting, the animals were anesthetize with isoflurane, and the 65 mg/mL STZ solution was administered to each animal from the tail vein with a volume of 1 mL/kg over about 15 seconds by using a 1 mL syringe and a 26G injection needle. After 14 days after the administration of STZ, body weight and pain response before drug administration were measured (see description below on pain response), and animals for each of which the 50% response threshold to mechanical stimulation was 6 g or lower in von Frey test were selected, and grouped so as to provide no difference in 50% response thresholds and body weight among groups.

The compound was prepared for oral administration in DMSO:1 mol/L HCl:Cremophor EL:0.5% methylcellulose = 10:5:5:80 to reach an administration volume of 10 mL/kg, and orally administered As a negative control, only the mixed solvent without the compound (Vehicle) was used.

Evaluation of pain response was performed by using von Frey test under blind conditions, without involving any measurer for pain response in grouping and administration. Measurement of 50% response thresholds by von Frey test was performed in accordance with a method by Chaplan et al. (J Neurosci Methods 1994; 53: 55-63) using von Frey filaments. Each animal was put in a measurement cage with a floor of a metal mesh about 30 minutes before scheduled measurement time, and left to stand to habituate to the environmental change, and after the animal was confirmed to become calm measurement was initiated. Avon Frey filament (bending force: 0.4, 0.6, 1, 2, 4, 6, 8, 15 g; starting from 2 g) was pressed vertically to the center of the sole of the left hind leg for about 6 seconds until the filament bent, and avoidance response (such as putting back the leg) was observed. In accordance with a Dixon's up-down method (Annu Rev Pharmacol Toxicol 1980; 20: 441-462), 50% response thresholds were calculated. Measurement was performed at four points of time: before drug administration on Day 14, and 30 minute, 90 minutes, and 180 minutes after the administration.

The results were represented as mean ± standard deviation. Dunnett's multiple comparison test was used for statistical analysis, and a significance level of 5% was used for both sides in all cases.

<Results>

The results of the STZ model are shown in Table 3. Significant analgesic effect due to pre-administration of the compound (I) was found. This result indicates the efficacy of the drug of the present application to animal models of SFN.

[Table 3]

| Group name | Number of cases | 50% response threshold (g) | | | |
|---|---|---|---|---|---|
| | | Day 14 | | | |
| | | Pre | 30 min | 90 min | 180 min |
| Vehicle | 10 | 3.6±0.4 | 4.7±0.9 | 4.9±1.2 | 4.5±0.6 |
| Compound (I) 0.6 mg/kg | 11 | 3.6±0.4 | 7.3±1.4 | 6.6±1.4 | 5.2±1.1 |
| Compound (I) 2 mg/kg* | 11 | 3.6±0.3 | 9.5±1.4 | 8.0±1.3 | 6.4±1.1 |
| Compound (I) 6 mg/kg*** | 11 | 3.6±0.4 | 12.5±1.1 | 12.1±1.5 | 9.6±1.4 |
| *P<0.05, ***P<0.001 vs. Vehicle (Dunnett's Test) | | | | | |

[Test Example 4] $dt^{sz}$ Hamster model

To confirm anti-dystoma effect in a dystonia model, a test with $dt^{sz}$ hamsters is conducted.

<Method>

Male $dt^{sz}$ hamsters (30-day- to 40-day-old), which undergo spontaneous onset of dystonia, are used for the test. The compound is prepared for oral administration in DMSO:1 mol/L HCl:Cremophor EL:0.5% methylcellulose = 10:5:5:80 to reach an administration volume of 10 mL/kg, and orally administered. As a negative control, only the mixed solvent without the compound (Vehicle) is used. Evaluation of dystonia is performed under blind conditions. Each hamster is put in a new plastic cage 2 days before evaluation of the compound, and observed for 3 hours to score dystonia developed in seven grades of 0 to 6 in accordance with Eur J Pharmacology 1993, 231, 287-291 (Pre-control). On the day of compound test, the compound (I) is orally administered with 0.6, 2, 6 mg/kg, and each hamster was put in a new plastic cage, and observed for 3 hours to score dystonia developed. Two days after that, an untreated hamster is put in a new plastic cage to score dystonia developed (Post-control), and individuals with score difference of 2 or more between the Pre-control value and the Post-control value are determined to have large variation and excluded. The significance of the anti-dystonia action by administration of the compound to the Vehicle administration group is examined through Wilcoxon signed-rank test

[Test Example 5] AD-related epilepsy model

To confirm anticonvulsant effect in an AD-related epilepsy model, a test with Tg2576 mice and 5XFAD mice is conducted.

<Method>

Tg2576 mice and 5XFAD mice are used for the test. The compound is prepared with a vehicle (10% DMSO, 0.05 mol/L HCl, 5% Cremophor EL, and 0.4% methylcellulose) to reach an administration volume of 10 mL/kg, and orally administered. Only the vehicle without the compound is used as a negative control. Evaluation of anticonvulsant effect is performed through measurement of convulsion thresholds or electroencephalography.

In a test to evaluate effect on convulsion thresholds, the compound or the vehicle is administered, and then convulsions are induced by corneal electrical stimulation. The presence or absence of convulsions after the intensity of the stimulation current is varied is visually determined, and the convulsion threshold is calculated in accordance with an up-down method (Kimball AW et al: Chemical protection against ionizing radiation. I. Sampling methods for screening compounds in radiation protection studies with mice. Radiat Res. 1957, 7(1), 1-12).

In a test to evaluate effect on spontaneous convulsive waves, animals with generation of a convulsive wave are selected out, and the compound or the vehicle is administered to each of these animals. The presence or absence of spontaneous convulsions before and after the administration is visually determined, and the electroencephalogram is quantitatively analyzed to evaluate the effect of the compound on convulsive waves.

[Test Example 6] Glioma-related epilepsy model

To confirm anticonvulsive effect in a glioma-related epilepsy model, a test with an intracranial transplantation model is conducted.

<Method>

Nude mice to which human glioma cells have been intracranially transplanted are used for the test. The compound is prepared with a vehicle (10% DMSO, 0.05 mol/L HCl, 5% Cremophor EL, and 0.4% methylcellulose) to reach an administration volume of 10 mL/kg, and orally administered. Only the vehicle without the compound is used as a negative control. The presence or absence of a convulsion wave is determined through electroencephalography. Animals with generation of a convulsive wave are selected out, and the compound or the vehicle is administered to each of these animals. The presence or absence of spontaneous convulsions before and after the administration is visually determined, and the electroencephalogram is quantitatively analyzed to evaluate the effect of the compound on convulsive waves.

[Test Example 7] Glioma model

To confirm antitumor effect in a glioma model, a test with a subcutaneous transplantation model is conducted.

<Method>

Nude mice to which human glioma cells have been subcutaneously transplanted are used for the test. The compound is prepared with a vehicle (10% DMSO, 0.05 mol/L HCl, 5% Cremophor EL, and 0.4% methylcellulose) to reach an administration volume of 10 mL/kg, and orally or intraperitoneally administered. Only the vehicle without the compound is used as a negative control. Antitumor effect is evaluated through quantitative analysis of tumor volume.

## Claims

1. An agent for treating essential tremor or small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

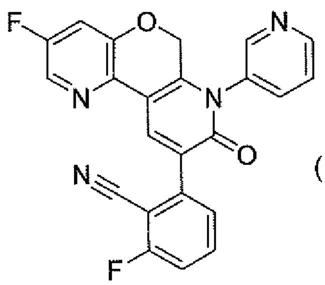

(I)

or a pharmaceutically acceptable salt thereof.

2. A method for treating essential tremor or small fiber neuropathy, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

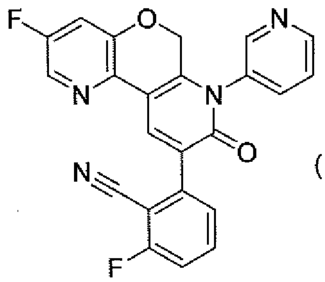

(I)

or a pharmaceutically acceptable salt thereof

**3.** 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

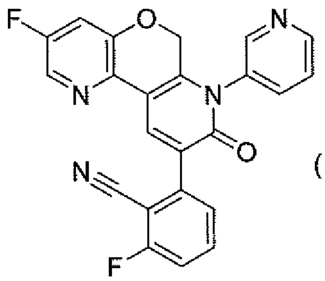

(I)

or a pharmaceutically acceptable salt thereof for use in treating essential tremor or small fiber neuropathy.

**4.** Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

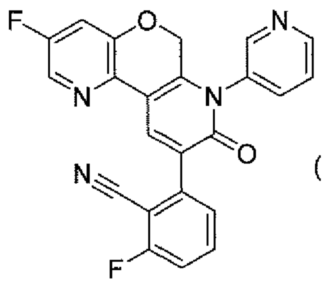

(I)

or a pharmaceutically acceptable salt thereof in the manufacture of an agent for treating essential tremor or small fiber neuropathy.

**5.** An agent for treating essential tremor, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

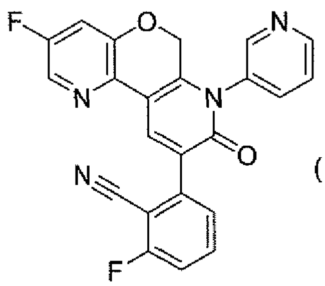

(I)

or a pharmaceutically acceptable salt thereof.

6. A method for treating essential tremor, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

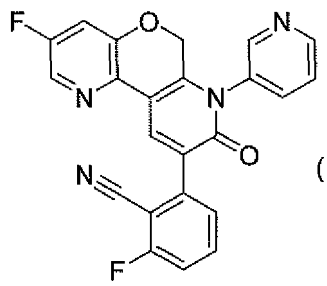

(I)

or a pharmaceutically acceptable salt thereof.

7. 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

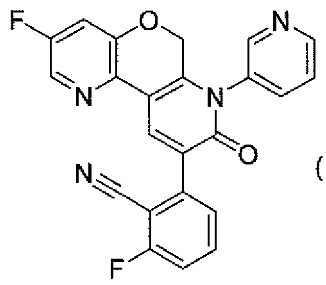

(I)

or a pharmaceutically acceptable salt thereof for use in treating essential tremor.

8. Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

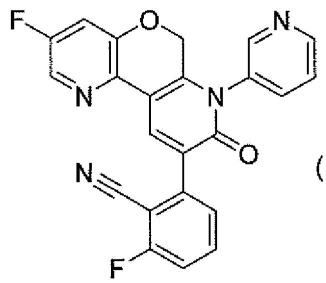

(I)

or a pharmaceutically acceptable salt thereof in the manufacture of an agent for treating essential tremor.

9. An agent for treating small fiber neuropathy, comprising 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

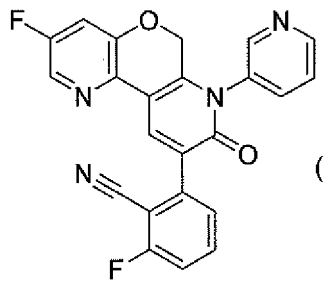

(I)

or a pharmaceutically acceptable salt thereof.

**10.** A method for treating small fiber neuropathy, comprising administering to a patient in need thereof 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

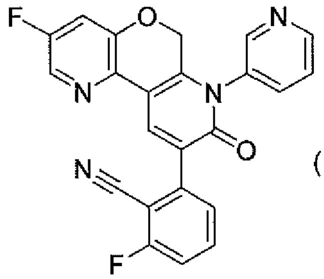

(I)

or a pharmaceutically acceptable salt thereof

**11.** 2-Fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

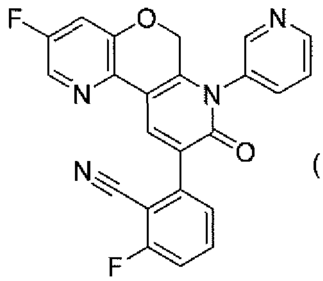

(I)

or a pharmaceutically acceptable salt thereof for use in treating small fiber neuropathy.

**12.** Use of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

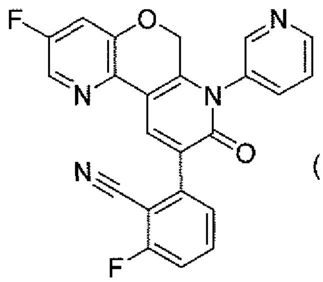

(I)

or a pharmaceutically acceptable salt thereof in the manufacture of an agent for treating small fiber neuropathy.

## INTERNATIONAL SEARCH REPORT

International application No. PCT/JP2018/017619

**A. CLASSIFICATION OF SUBJECT MATTER**

Int.Cl. A61K31/444(2006.01)i, A61P25/14(2006.01)i, A61P43/00(2006.01)i, C07D491/147(2006.01)i

According to International Patent Classification (IPC) or to both national classification and IPC

**B. FIELDS SEARCHED**

Minimum documentation searched (classification system followed by classification symbols)
Int.Cl. A61K31/444, A61P25/14, A61P43/00, C07D491/147

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY/MEDLINE/EMBASE/BIOSIS/WPIDS (STN) JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2012-515192 A (NEUROSEARCH A/S) 05 July 2012, paragraphs [0112], [0113] & US 2012/0015977 A1, paragraphs [0148], [0149] & WO 2010/081900 A1 & EP 2387562 A1 | 1-12 |
| A | PATERSON, NE et al., "Pharmacological characterization of harmaline-induced tremor activity in mice", Eur. J. Pharmacol., 2009, vol. 616, pp. 73-80 | 1-12 |
| A | TOMIYAMA, M. et al., "Upregulation of mRNAs coding for AMPA and NMDA receptor subunits and metabotropic glutamate receptors in the dorsal horn of the spinal cord in a rat model of diabetes melli tus", Mol. Brain Res., 2005, vol. 136, pp. 275-281 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>20 July 2018 (20.07.2018) | Date of mailing of the international search report<br>07 August 2018 (07.08.2018) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

International application No. PCT/JP2018/017619

C (Continuation). DOCUMENTS CONSIDERED TO BE RELEVANT

| Category* | Citation of document, with indication, where appropriate, of the relevant passages | Relevant to claim No. |
|---|---|---|
| A | HIBI, S. et al., "Discovery of 2-(2-0xo-1-pheny1-5-pyridin-2-y1-1, 2-dihydropyridin-3-y1)benzoni tri le (Perampanel): A novel, noncompetitive α-amino-3-hydroxy-5-methy1-4-isoxazolepropanoic acid (AMPA) receptor antagonist", J. MED. CHEM., 2012, vol. 55, pp. 10584-10600 | 1-12 |
| P, X<br>P, A | WO 2017/082288 A1 (EISAI R&D MANAGEMENT CO., LTD.) 18 May 2017, claim 1(XIV) & US 2017/0137436 A1, claim 1(XIV) & TW 201720829 A & AR 106647 A | 3, 7, 11<br>1, 2, 4-6, 8-10, 12 |

Form PCT/ISA/210 (continuation of second sheet) (January 2015)

**INTERNATIONAL SEARCH REPORT**
Information on patent family members

International application No.

Form PCT/ISA/210 (patent family annex) (January 2015)

**INTERNATIONAL SEARCH REPORT**

International application No.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Neurology,* 2005, vol. 64, 2008-2020
- *Parkinsonism and Related Disorders,* 2010, vol. 16, 604-607
- *Journal of Central Nervous System Disease,* 2013, vol. 5, 43-55
- *The American Journal of Medicine,* 2003, vol. 115, 134-142
- *Journal of Neurological Sciences,* 2015, vol. 349, 168-173
- *Journal of Physiology,* 1986, vol. 376, 163-182
- *European Journal of Pharmacology,* 2009, vol. 616, 73-80
- **MARTYN CN et al.** *Neurol Neurosurg Psychiatry Epidemiology of peripheral neuropathy,* 1997
- **HUGHES RC et al.** Peripheral Neuropathy. *Br Med J,* 2002, vol. 324, 466-469
- **BASBAUM AI et al.** Cellular and molecular mechanisms of pain. *Cell,* 2009, vol. 139 (2), 267-84
- **CHAN AC ; WILDER-SMITH EP.** Small fiber neuropathy: Getting bigger!. *Muscle Nerve,* 2016, vol. 53 (5), 671-82
- **TAVEE J ; ZHOU L.** *Cleve Clin J Med.,* 2009, vol. 76 (5), 297-305
- **BAKKERS M et al.** Small fibers, large impact: quality of life in small-fiber neuropathy. *Muscle Nerve,* 2014, vol. 49 (3), 329-36
- Management of painful neuropathies. **NANNA BRIX FINNERUP et al.** Handbook of Clinical Neurology. 2013, vol. 115, 279-290
- **VIKTOR R DREL et al.** Evaluation of the peroxynitrite decomposition catalyst Fe(III) tetramesitylporphyrin octasulfonate on peripheral neuropathy in a mouse model of type 1 diabetes. *Int J Mol Med.,* 2007, vol. 20 (6), 783-792
- **TOMIYAMA M et al.** Upregulation of mRNAs coding for AMPA and NMDA receptor subunits and metabotropic glutamate receptors in the dorsal horn of the spinal cord in a rat model of diabetes mellitus. *Brain Res Mol Brain Res,* 2005, vol. 20; 136 (1-2), 275-281
- **BRESSMAN SB.** Dystonia genotypes, phenotypes, and classification. *Adv Neurol,* 2004, vol. 94, 101-107
- **PAGE D et al.** Quality of life in focal, segmental, and generalized dystonia. *Mov Disord,* 2007, vol. 22 (3), 341-347
- **E. ZOONS et al.** Botulinum toxin as treatment for focal dystonia: a systematic review of the pharmaco-therapeutic and pharmaco-economic value. *Journal of Neurology,* 2012, vol. 259 (12), 2519-2526
- **TERMSARASAB P et al.** Medical treatment of dystonia. *J Clin Move Disorder,* 2016, vol. 3 (19
- **RICHTERA et al.** *The AMPA receptor antagonist NBQX exerts antidystonic effects in an animal model of idiopathic dystonia,* 1993, vol. 231, 287-291
- **HORVATH A et al.** Epileptic seizures in Alzheimer disease: A review. *Alzheimer Dis. Assoc. Disord.,* 2016, vol. 30 (2), 186-192
- **VOSSEL KA et al.** Incidence and impact of subclinical epileptiform activity in Alzheimer's disease. *Ann. Neurol.,* 2016, vol. 80 (6), 858-870
- **ZAREA A et al.** Seizures in dominantly inherited Alzheimer disease. *Neurology,* 2016, vol. 87 (9), 912-919
- **JOUTSA J et al.** Association between childhood-onset epilepsy and amyloid burden 5 decades later. *JAMANeurol,* 2017, vol. 74 (5), 583-590
- **MARCELLO E et al.** SAP97-mediated local trafficking is altered in Alzheimer disease patients' hippocampus. *Neurobiol. Aging,* 2012, vol. 33 (2), 422.e1-10
- **GAISLER-SALOMON I et al.** Hippocampus-specific deficiency in RNAediting of GluA2 in Alzheimer's disease. *Neurobiol. Aging,* 2014, vol. 35 (8), 1785-1791
- **WHITCOMB DJ et al.** Intracellular oligomeric amyloid-beta rapidly regulates GluA1 subunit ofAMPA receptor in the hippocampus. *Sci. Rep.,* 2015, vol. 5, 10934
- **BEZZINA C et al.** Early onset of hypersynchronous network activity and expression of a marker of chronic seizures in the Tg2576 mouse model of Alzheimer's disease. *PLoS One,* 2015, vol. 10 (3), e0119910
- **SIWEK ME et al.** Altered theta oscillations and aberrant cortical excitatory activity in the 5XFAD model of Alzheimer's disease. *Neural Plast,* 2015, 2015
- **LI Y et al.** IDH1 mutation is associated with a higher preoperative seizure incidence in low-grade glioma: A systematic review and meta-analysis. *Seizure,* 2018, vol. 55, 76-82
- **NEAL A et al.** Postoperative seizure control in patients with tumor-associated epilepsy. *Epilepsia,* 2016, vol. 57 (11), 1779-1788

- **BUCKINGHAM SC et al.** Glutamate release by primary brain tumors induces epileptic activity. *Nat Med.,* 2011, vol. 17 (10), 1269-1274
- **ISHIUCHI S et al.** Blockage of Ca2+-permeable AMPA receptors suppresses migration and induces apoptosis in human glioblastoma cells. *Nat. Med,* 2002, vol. 8 (9), 971-978
- United States Pharmacopeia, and the European Pharmacopoeia. General Rules for Preparations of the Japanese Pharmacopoeia
- *Journal of the American Chemical Society,* 2011, vol. 133 (35), 14082-14089
- *CHEMICAL ABSTRACTS,* 67226-78-2
- *Journal of Medicinal Chemistry,* 2008, vol. 51 (15), 4708-4714
- *CHEMICAL ABSTRACTS,* 177559-01-2
- *CHEMICAL ABSTRACTS,* 163080-99-7
- *CHEMICAL ABSTRACTS,* 149-73-5
- *CHEMICAL ABSTRACTS,* 623942-84-7
- *CHEMICAL ABSTRACTS,* 660425-16-1
- *CHEMICAL ABSTRACTS,* 131534-65-1
- *CHEMICAL ABSTRACTS,* 765916-91-4
- *CHEMICAL ABSTRACTS,* 591-55-9
- *CHEMICAL ABSTRACTS,* 40052-13-9
- *CHEMICAL ABSTRACTS,* 39889-94-6
- *Neuroscience Letter,* 2015, vol. 590, 84-90
- **CHAPLAN et al.** *J Neurosci Methods,* 1994, vol. 53, 55-63
- *Annu Rev Pharmacol Toxicol,* 1980, vol. 20, 441-462
- *Eur J Pharmacology,* 1993, vol. 231, 287-291
- **KIMBALL AW et al.** Chemical protection against ionizing radiation. I. Sampling methods for screening compounds in radiation protection studies with mice. *Radiat Res.,* 1957, vol. 7 (1), 1-12